**Europäisches Patentamt**

**European Patent Office**

**Office européen des brevets**

(19)

(11) Veröffentlichungsnummer : **0 380 085 B1**

(12) # EUROPÄISCHE PATENTSCHRIFT

(45) Veröffentlichungstag der Patentschrift :
**02.01.92 Patentblatt 92/01**

(51) Int. Cl.⁵ : **C07F 13/00,** C07D 301/12,
B01J 31/02

(21) Anmeldenummer : **90101439.9**

(22) Anmeldetag : **25.01.90**

(54) **Verwendung von rheniumorganischen Verbindungen zur Oxidation von C-C-Mehrfachbindungen, darauf basierende Oxidationsverfahren und neue rheniumorganische Verbindungen.**

(30) Priorität : **27.01.89 DE 3902357**

(43) Veröffentlichungstag der Anmeldung :
**01.08.90 Patentblatt 90/31**

(45) Bekanntmachung des Hinweises auf die
Patenterteilung :
**02.01.92 Patentblatt 92/01**

(84) Benannte Vertragsstaaten :
**BE CH DE FR GB IT LI NL**

(56) Entgegenhaltungen :
EP-A- 0 308 791
US-A- 4 418 203
ANGEWANDTE CHEMIE, 100. Jahrgang, 1988,
Weinheim W.A. HERMANN et al.
"Methylrheniumoxide: Synthese aus Re207
und Katalyse- aktivitUt in der Olefin- metathese" Seiten 420-422

(73) Patentinhaber : **HOECHST
AKTIENGESELLSCHAFT
Postfach 80 03 20
W-6230 Frankfurt am Main 80 (DE)**

(72) Erfinder : **Herrmann, Wolfgang Anton, Prof. Dr.
Waldweg 10
W-8051 Giggenhausen (DE)**
Erfinder : **Marz, Dieter
Fritz-Meyer-Weg 55
W-8000 München 81 (DE)**
Erfinder : **Wagner, Werner
Schlierseestrasse 29
W-8000 München 90 (DE)**
Erfinder : **Kuchler, Josef G.
Rathgeberstrasse 16
W-8000 München 50 (DE)**
Erfinder : **Weichselbaumer, Georg
Richildisstrasse 6
W-8899 Hohenwart (DE)**
Erfinder : **Fischer, Richard
Karolinenstrasse 7
W-8201 Grosskarolinenfeld (DE)**

## Beschreibung

Die Erfindung bezieht sich auf die Verwendung von bestimmten rheniumorganischen Verbindungen zur Oxidation von C-C-Mehrfachbindungen, darauf basierende Oxidationsverfahren, insbesondere zur Herstellung von Epoxiden und gegebenenfalls Diolen durch Oxidation von olefinischen Verbindungen bzw. zur Herstellung von Diketonen bzw. $\alpha,\beta$-olefinisch ungesättigten Monoketonen durch Oxidation von Alkinen, jeweils mit Wasserstoffperoxid mittels rheniumorganischer Verbindungen, und auf neue rheniumorganische Verbindungen.

Es ist bekannt, daß Metalloxide selektiv wirksame Oxidationsmittel für ungesättigte organische Verbindungen unterschiedlicher Substanzklassen sind. So werden in der Praxis insbesondere die binären Oxide $M_xO_y$ der Metalle (M) Vanadium ($V_2O_5$), Molybdän ($MoO_3$) und Osmium ($OsO_4$) beispielsweise als Katalysatoren zur Überführung von Olefinen in Epoxide eingesetzt. Dabei können auch 1,2-Diol-Derivate entstehen, im Falle von $OsO_4$ stereospezifisch die cis-Glykole. Häufig werden diese Metalloxide nicht als stöchiometrische Reagenzien, sondern in Gegenwart von Wasserstoffperoxid, tert.-Butylhydroperoxid, m-Chlorperbenzoesäure usw. in nur katalytischen Mengen eingesetzt. Obwohl die Reaktionsmechanismen im einzelnen unbekannt sind, diskutiert man für die Wirkungsweise solcher Katalysatorsysteme (z.B. Metalloxid/$H_2O_2$) sog. Hydroperoxo-Metallkomplexe als eigentlich wirksame Katalysatorspezies (Struktureinheit M-O-OH).

Eine besondere Bedeutung für die selektive Oxidation von Olefinen zu cis-1,2-Diolen besitzt das Katalysatorsystem $OsO_4/H_2O_2$. In diesem Fall reichen katalytische Mengen des Osmiumtetroxids aus. Besonders bekannt ist "Milas' Reagenz" : $H_2O_2$/tert.-Butanol/$OsO_4$ (N. A. Milas und S. Sussaman, J. Am. Chem. Soc. 58 (1936) 1302-04 und N. A. Milas und S. Sussaman J. Am. Chem. Soc. 59 (1937) 2345-47).

Die oben genannten Metalloxide weisen in diesem Zusammenhang allerdings auch Nachteile auf, von denen der hohe Preis (Osmium), die Hohe Toxizität (Osmium), die fehlende Selektivität für die Bildung von Epoxiden und cis-1,2-Diolen aus den olefinischen Vorstufen (Chrom, Molybdän, Wolfram) und die Kurzlebigkeit der Katalysatoren unter den gegebenen Reaktionsbedingungen (insbesondere $OsO_4/H_2O_2$/tert.-Butanol) besonders hervorzuheben sind.

Es bestand daher die Aufgabe, ein möglichst leicht zugängliches, einfach handhabbares, lagerfähiges, ungiftiges und wirksames Katalysatorsystem zu finden, das die gewünschte Selektivität bei der Oxidation von Olefinen und bestimmten Alkinen erreicht. Den 1,2-Diolen und Epoxiden kommt für die industrielle Weiterverarbeitung großes Interesse zu, aber auch den 1,2-Diketonen bzw. $\alpha,\beta$-olefinisch ungesättigen Monoketonen.

Die naheliegende Vorstellung, daß anstelle der literaturbekannten Oxidationskatalysatoren auch Oxide des den Elementen Osmium und Molybdän im Periodensystem nahestehenden Elements Rhenium, das ja um ein Vielfaches billiger ist als Osmium, der Formel $Re_xO_y$ eingesetzt werden könnten, hat sich experimentell nicht bestätigen lassen. So sind die Oxide $Re_2O_7$, $ReO_3$ und $Re_2O_5$ in Abwesenheit und auch in Gegenwart anderer Oxidationsmittel wie Wasserstoffperoxide und tert.-Butylhydroperoxid bei der Oxidation von Olefinen entweder inaktiv oder unselektiv. In der Literatur finden sich nur zwei, einander sehr ähnliche Angaben über die Verwendung von Rheniumverbindungen, und zwar Rheniumoxiden, Rheniumhalogeniden und Perrhenaten, als Katalysatoren für die Oxidation mit Sauerstoff bei −50 bis +125°C (US-PS 3,316,279) bzw. mit Wasserstoffperoxid (EP-A-0308791) bei 100-150°C (US-PS 3,518,285). Dabei wurden stets Gemische von Epoxiden und Ketonen erhalten. Bei der Oxidation mit Sauerstoff gemäß US-PS 3,316,279 können auch "Oxidations-Modifizierungsmittel", und zwar stickstoffhaltige Verbindungen, nämlich organische Cyanide, Pyridine und Chinoline mitverwendet werden, die die Reaktion bis zu einem gewissen Grade auf der Stufe der Epoxide zum Stehen bringen und Umlagerungen zurückdrängen sollen ; in ihrer Abwesenheit treten Folgereaktionen wie die Bildung von Kondensationsprodukten (durch die Dimerisation der Epoxide zu substituierten Dioxanen oder Polymerisation zu Polyäthern) und die Bildung von $\alpha$-Hydroxycarbonylverbindungen durch weitere Oxidation der Epoxide ein. Außerdem soll das Verfahren gemäß der US-PS 3,316,279 bei Drucken von 1 bis etwa 250 Atmosphären durchgeführt werden, d.h. es ist praktisch das Arbeiten bei erhöhtem Druck obligatorisch, wie sich auch aus den Beispielen ergibt. Druckreaktionen erfordern aber einen erhöhten technischen Aufwand.

Eigene Versuche, die oben genannten Rheniumoxide, z.B. $Re_2O_7$ und $ReO_3$, als Katalysatoren zur Olefinoxidation zu verwenden, waren ebenfalls erfolglos. Auch $Na[ReO_4]$, $NH_4[ReO_4]$ und $[N(n\text{-}C_4H_9)_4][ReO_4]$ erwiesen sich als unwirksam, und ebenso auch metallorganische Mischverbindungen, wie das von W.A. Herrmann et al. beschriebene monomere Derivat (Trimethylstannoxy)rheniumtrioxid der Formel $[(CH_3)_3SnO]ReO_3$ (W.A. Herrmann, J. G. Kuchler, J. K. Felixberger, E. Herdtweck und W. Wagner, Angew. Chem. 100 (1988) 420-422) und die Verbindungen $[(CH_3)_3SiO]ReO_3$ (M. Schmidt und H. Schmidbaur, Chem. Ber. 92 (1959) 2667-2669) und $[(C_6H_5)_3SiO]ReO_3$ (H. Schmidbaur und D. Koth, Chem. Zeitung 100 (1976) 290-291).

Von den echten metallorganischen Verbindungen des Rheniums ist aus der Arbeit von W.A. Herrmann et al., l.c. bekannt, daß Methylrheniumtrioxid $CH_3ReO_3$ in Gegenwart von $AlCl_3$ und Tetramethylzinn die Metathese offenkettiger Olefine und auch die katalytische Ringöffnungspolymerisation von Cycloolefinen zu Polyalkenameren bewirkt. Aus der gleichen Veröffentlichung ist bekannt, daß das Methylrheniumoxid bei der Behandlung mit Dimethylzink zu einem sublimierbaren, zitronengelben Methylrheniumoxid der Formel $(CH_3)_4Re_2O_4$ methyliert wird, also zu einer Verbindung mit 6-wertigem Rhenium. Auch diese Verbindung katalysiert in Gegenwart von Tetramethylzinn und Aluminiumchlorid die metathetische Ringöffnungspolymerisation von Cyclopenten.

Aus der genannten Veröffentlichung ist schließlich bekannt, daß das $(CH_3)_4Re_2O_4$ durch weitere Methylierung mit Dimethylzink zu $(CH_3)_6Re_2O_3$ umgesetzt wird, also ebenfalls zu einer Verbindung mit 6-wertigem Rhenium. Diese Verbindung ist nach der genannten Literaturstelle in der Lage, die Ringöffnungspolymersiation von Cyclopenten selbst dann zu katalysieren, wenn nur $AlCl_3$, nicht aber der sonst noch notwendige Cokatalysator $Sn(CH_3)_4$ zugegen ist.

Aus diesen Angaben und der übrigen Literatur konnte keinesfalls geschlossen werden, daß rheniumorganische Verbindungen auch für die gänzlich andersartige Reaktion der Oxidation von C-C-Mehrfachbindungen katalytisch wirken, und zwar ohne Cokatalysator.

Es wurde nun überraschend gefunden, daß bestimmte organische Verbindungen des Rheniums als hochaktive Katalysatoren für die Oxidation von C-C-Mehrfachbindungen, insbesondere für die selektive Überführung von Olefinen in 1,2-Diole oder Epoxide, geeignet sind, wenn sie zusammen mit Wasserstoffperoxid in einem flüssigen Medium angewendet werden.

Gegenstand der Erfindung ist nun die Verwendung von Verbindungen der allgemeinen Formel $R^1_aRe_bO_c$ (I), worin a 1 bis 6, b 1 bis 4 und c 1 bis 14 sind und die Summe von a, b und c so ist, daß sie der 5- bis 7-Wertigkeit des Rheniums gerecht wird mit der Maßgabe, daß c nicht größer als $3 \cdot b$ ist, und worin $R^1$ gleich oder verschieden und zwar einen nicht-aromatischen Kohlenwasserstoffrest mit 1 bis 10 C-Atomen oder Aralkyl mit 7 bis 9 C-Atomen darstellt, wobei $R^1$ wenigstens teilweise fluoriert sein kann, die Verbindungen nicht mehr als drei Gruppen mit mehr als 6 C-Atomen je Rheniumatom enthalten und an das C-Atom in $\alpha$-Stellung noch wenigstens ein Wasserstoffatom gebunden ist, bzw. von neuen Verbindungen der Formel $(R^{11}Re)_kO_lR^{12}_m \cdot L_n$ (III), worin $R^{11}$, $R^{12}$, L, k, l, m und n die unter angegebene Bedeutung haben, bzw. von neuen Verbindungen der Formel $(Polymer)_f \cdot (CH_3ReO_3)_g$ (VI), in der die Rheniumverbindung $CH_3ReO_3$ über Amin- oder Amidstickstoff an das Polymere gebunden ist und in der der Quotient g/f das Gewichtsverhältnis der beiden Komponenten wiedergibt und im Bereich zwischen 0,01 und 0,2, vorzugsweise zwischen 0,02 und 0,1 liegt, als Katalysatoren zur Oxidation von C-C-Mehrfachbindungen.

Gegenstand der Erfindung sind auch Verbindungen der Formel $(R^{11}Re)_kO_lR^{12}_m \cdot L_n$ (III) worin, bedeuten

$R^{11}$ einen nichtaromatischen Kohlenwasserstoffrest mit 1 bis 10 C-Atomen oder Aralkyl mit 7-9 C-Atomen, die jeweils durch ein Kohlenstoffatom, an das noch wenigstens ein Wasserstoffatom gebunden ist, an das Rhenium gebunden sind, und zwar vorteilhaft Alkylreste mit 1 bis 9 C-Atomen und Cycloaklyl mit 5 bis 10 C-Atomen,

$R^{12}$ einen linearen oder verzweigten aliphatischen Kohlenwasserstoffrest mit 1 bis 10 C-Atomen, der auch bis zu 3 Siliziumbrückenatome enthalten kann, oder einen Phenylrest, der unsubstituiert ist oder bis zu 3 Substituenten aus der Gruppe Alkyl und/oder Alkoxy mit zusammen höchstens 6 C-Atomen oder bis zu fünf Fluoratome enthält,

L einen Liganden, der über ein bis drei O- und/oder N-Atome an das Rhenium gebunden ist, und die

Indizes k 1 oder 2, l eine ganze Zahl von 1 bis $3 \cdot k$, m null oder eine ganze Zahl von 1 bis $2 \cdot k$ und n eine ganze Zahl von 1 bis $3 \cdot k$.

Die Indizes k, l, m und n müssen so gewählt sein, daß ihre Kombination der 5-, 6- oder 7-Wertigkeit des Rhenium gerecht wird ; der Ligand K kann 1-, 2- oder 3-zähnig sein und kann für sich genommen neutral oder anionisch sein.

In den Formeln I und III bedeuten $R^1$ und $R^{11}$ einen nicht-aromatischen Kohlenwasserstoffrest mit 1 bis 10 C-Atomen oder Aralkyl mit 7 bis 9 C-Atomen, wie Benzyl, die jeweils durch ein Kohlenstoffatom, an das noch wenigstens ein Wasserstoffatom gebunden ist, an das Rhenium gebunden sind, und zwar Alkylreste mit 1 bis 10 C-Atomen oder Cycloalkyl mit 5 bis 10 C-Atomen. Geeignet sind Alkylreste wie Methyl, Äthyl, Propyl, Isopropyl und die verschiedenen Butyl-, Pentyl-, Hexyl-, Octylreste wie Äthylhexyl- und Decylreste ; geeignet sind auch Cycloalkylreste wie Cyclopentyl, Cyclohexyl, alkyliertes Cyclohexyl wie hydriertes Toluyl, Xylyl, Äthylphenyl, Cumyl oder Cymyl und 1-Norbornyl. Methyl ist besonders bevorzugt.

Die Begriffe Alkyl und Cycloalkyl beinhalten naturgemäß, daß die Gruppen keine mehrfachbindungen enthalten. $R^1$ kann wenigstens teilweise fluoriert sein. Jedoch kommt in den Verbindungen I und III aus sterischen Gründen die Anwesenheit von mehr als drei Gruppen mit mehr als 6 C-Atomen je Rheniumatom nicht in Betracht ; zweckmäßig enthalten die Verbindungen nur höchstens eine solche Gruppe.

Gegenstand der Erfindung sind auch die vorgenannten Verbindungen der Formel $(Polymer)_f \cdot (CH_3ReO_3)_g$ (VI).

Gegenstand der Erfindung ist auch ein Verfahren zur Herstellung von Epoxiden durch Oxidation von olefinischen Verbindungen mit Wasserstoffperoxid in Gegenwart von Katalysatoren, und gegebenenfalls weitere Umsetzung zu Diolen, das dadurch gekennzeichnet ist, daß man Olefine der Formel $YCZ=CZ-(CX_2)_nR^2$ (II),

worin $R^2$, X, Y, Z und n die unten genannten Bedeutungen haben, bei einer Temperatur von −30 bis +80° C, vorzugsweise von 0 bis 40° C, in flüssigem Medium in Gegenwart von Rhenium-Verbindungen der oben genannten Formel $R^1_a Re_b O_c$ (I), worin $R^1$, a, b und c die oben genannten Bedeutungen haben, bzw. von Verbindungen der Formel $(Re^{11}Re)_k O_l R^{12}_m \cdot L_n$ (III) bzw. von Verbindungen der Formel $(Polymer)_f \cdot (CH_3ReO_3)_g$ (VI), wie zuvor definiert, oxidiert und die erhaltenen Epoxide

    a) isoliert order

    b) in Gegenwart der gleichen Katalysatoren in 1,2-Diole oder

    c) in üblicher Weise mit Basen in 1,2-trans-Diole oder

    d) in üblicher Weise mit Säuren in cis-trans-Gemische von 1,2-Diolen umwandelt.

In der Formel II stellt n 0 oder eine ganze Zahl von 1 bis 28 dar ; X ist H oder F ; Y ist H, Alkyl oder ein einfach oder mehrfach olefinisch-ungesättigter, unverzweigter oder verzweigter, offenkettiger oder cyclischer Kohlenwasserstoffrest mit 2 bis 28 C-Atomen, z.B. Alkyl mit 1 bis 10 C-Atomen oder Aryl mit 6 bis 10 C-Atomen, oder $COOR^3$. Z stellt H oder einen aromatischen oder nichtaromatischen Kohlenwasserstoffrest mit 1 bis 6 C-Atomen dar, der cyclisch sein kann, z.B. Cyclohexyl ist, vorzugsweise aber offenkettiges Alkyl mit 1 bis 4 C-Atomen ist. Beide Z können auch gleichzeitig CO darstellen, die in o-Stellung an einen Benzolrest gebunden sind, wie im Naphthochinon, oder zusammen mit C=C einen cycloaliphatischen Ring bilden, wie im Cyclohexen, 1-Methyl-1-Cyclohexen, Cyclohepten, Cycloocten, Cyclooctadien, Cyclooctatetraen, Cyclododecen, Cyclohexadecadien oder Limonen. Die Substituenten Z können gleich oder verschieden sein. $R^2$ bedeutet H, Halogen, einen acyclischen, von Dreifachbindungen freien Kohlenwasserstoffrest mit bis zu 24 C-Atomen, der bis zu 5 Doppelbindungen, enthalten kann, wie im Squalen, Aryl mit bis zu 10 C-Atomen, das am Ring 1 bis 3 Substituenten enthalten kann, die unter den angewandten Oxidationsbedingungen unempfindlich sind, ω-Oxoalkenyl, $COOR^3$ oder $OR^4$, wobei $R^3$ Alkyl oder Aryl und $R^4$ Alkyl, Aryl oder Trialkylsilyl $R^5_3 Si$ ist. Die Alkylgruppen in $R^5$ enthalten 1 bis 5, vorzugsweise 1 bis 3 C-Atome. In $R^3$ und $R^4$ steht Alkyl jeweils für solches mit 1 bis 15, vorzugsweise 1 bis 6 Kohlenstoffatomen, und Aryl für Phenyl, das am Ring noch ein bis drei Substituenten enthalten kann, die unter den angewandten Oxidationsbedingungen inert sind, wie Halogen, z.B. Fluor, Chlor oder Brom, $NO_2$, $OR^6$ und/oder Alkyl. Die Reste $R^6$ sind gleich oder verschieden und können Wasserstoff oder Alkyl mit 1 bis 4 C-Atomen sein. $(CX_2)_n$ und $R^2$ haben zusammen höchstens 28 C-Atome. Y, $(CX_2)_n$ und $R^2$ können zusammen mit der Gruppe CZ=CZ auch ein cycloaliphatisches Ringsystem bilden, das bis zu 5, vorzugsweise bis zu 3 olefinische Bindungen oder bis zu zwei Carbonylgruppen enthalten kann. Solche Ringsystem liegen z.B. im Pinen und Dipenten (Limonen) vor.

$R^2$ als Halogen kann Fluor, Chlor, Brom oder Jod sein. Wenn Z Wasserstoff und $R^2$ Halogen ist, ist dieses bevorzugt Brom. Verbindungen, in denen wenigstens ein X=F ist, sind z.B. 4-(Perfluor-n-hexyl)buten (1) der Formel $(n-C_6F_{13})-CH_2-CH_2-CH=CH_2$ und Perfluorpropen $C_3F_6$. Die Gliederzahl n variiert vorzugsweise im Bereich von 1 bis 12 und insbesondere bis 8.

Gegenstand der Erfindung ist auch ein Verfahren zur Oxidation von Cumulenen der Formel $R^7C=C=CR^8$ (IV) und von Alkinen der Formel $R^9C≡CR^{10}$ (V), worin $R^7$ bis $R^{10}$ jeweils Alkyl mit 1 bis 4 C-Atomen oder 1 oder 2 Arylkerne enthaltendes Aryl ist, wie Naphthyl, Biphenyl oder insbesondere Phenyl, wobei das Aryl jeweils gleich oder verschieden sein und noch Substituenten tragen kann, die ihrerseits unter den angewandten Oxidationsbedingungen unempfindlich sind. Dabei bilden sich aus den Cumulenen Hydroxyverbindungen, deren Hydroxylgruppe am wasserstoffärmsten C-Atom steht, und aus Alkinen 1,2-Diketone oder, wenn wenigstens einer der Reste $R^9$ und $R^{10}$ an die acetylenische Gruppe durch eine CH-Gruppierung gebunden ist, α,β-olefinisch ungesättigte Monoketone. Diese Oxidationen werden ebenfalls mittels Rhenium-Verbindungen der Formeln I, III und VI wie oben definiert, mit Wasserstoffperoxid bei einer Temperatur von −30 bis +80°C, vorzugsweise von 0-40°C, in flüssigem Medium durchgeführt.

Nach einer weiteren Ausführungsform der Erfindung lassen sich auch Verbindungen der Formel $R^{13}HC=CHR^{14}$ (VII), in denen $R^{13}$ Aryl mit bis zu 10 C-Atomen und $R^{14}$ H, $CH_3$ oder Aryl mit bis zu 10 C-Atomen darstellt und in denen die Arylreste gleich oder verschieden sind, mit Wasserstoffperoxid bei einer Temperatur von −30 bis +80°C, vorzugsweise von 0-40°C, in flüssigem Medium in Gegenwart von Rhenium-Verbindungen der Formeln I, III bzw. VI, wie oben definiert oxidieren. Dabei werden hauptsächlich Aldehyde gebildet. Geeignete Ausgangsmaterialien für diese Ausführungsform der Erfindung sind z.B. Styrol, die verschiedenen Vinyltoluole und Stilben. Auch hier kann das Aryl noch Substituenten tragen, die unter den angewandten Oxidationsbedingungen unempfindlich sind, z.B. 2,3,4,5,6-Pentafluorstyrol.

Die Verbindungen der Formel I sind z.T. bekannt (W.A. Herrmann et al. Angew. Chem. 100 (1988) 420-422), z.T. Gegenstand der älteren europäischen Patentanmeldung P 89122437.0 ; ihre Eignung als Oxidationskatalysatoren ist jedoch neu und war nicht zu erwarten. Sie sind vielmehr die ersten metallorganischen Verbindungen des Rheniums, die für die Ausführung von Oxidationsverfahren erfolgreich eingesetzt werden können. Wegen ihrer Löslichkeitseigenschaften eignen sie sich vorzüglich als Homogenkatalysatoren. Ihr besonderer Vorteil liegt auch darin, daß sie auf einfache Weise aus handelsüblichem $Re_2O_7$ mit Hilfe gängiger,

als Überträger von organischen Gruppen wirkender Substanzen synthetisiert werden können, z.B. im Falle von $R^1 = CH_3$ durch Umsetzung mit handelsüblichem Tetramethylzinn oder handelsüblichem Dimethylzink. Sie sind gegenüber Luft und Feuchtigkeit, Wasser und Säure unempfindlich, bei Raumtemperatur lagerfähig und in Kombination mit Wasserstoffperoxid, anorganischen Peroxiden wie Alkaliperoxiden, insbesondere Natriumperoxid, sowie Percarbonsäuren und ihren Salzen wie m-Chlorperbenzoesäure, Peressigsäure und Magnesiummonoperoxophthalat hochaktive Katalysatoren für die erfindungsgemäßen Oxidationen.

Die Verbindungen der Formel III unterscheiden sich von denen der Formel $R^1{}_a Re_b O_c$ (I) wesentlich dadurch, daß sie zusätzlich Stickstoffbasen und/oder Oxid- oder Oxo- Sauerstoff bzw. Hydroxyl enthalten, die komplexartig an das Rhenium gebunden sind. Im Rest $R^{12}$ kann Alkyl z.B. Methyl, Äthyl, Propyl, einen der Butyl-, Pentyl-, Hexyl-, Octyl- oder Decylreste bedeuten. Als substituierter Phenylrest sei z.B. der Tolyl-, Xylyl- oder Anisylrest genannt.

Der Ligand ist z.B. ein Amin wie Ammoniak, ein primäres Amin $H_2NR$, sekundäres Amin $HNR_2$ oder tertiäres Amin $NR_3$, worin R verzweigtes oder unverzweigtes Alkyl mit 1-18 Kohlenstoffatomen bedeuten kann, ein aliphatischer Azacyclus wie Chinuclidin, ein aromatischer Azacyclus, wie Pyridin und seine ringsubstituierten und anellierten Derivate, wie 8-Hydroxychinolin, ein O,O'-, N,O- oder N,N'-Chelatligand wie 2,2'-Bipyridin, 2-Aminopyridin, 2-(Aminomethyl)pyridin, ein substituiertes Piperazin, ein aminosubstituiertes Piperidin oder Pyrrolidin, ein methoxysubstituiertes Pyridin wie 2-Methoxypyridin, ein 1,3-Diketon wie Acetylaceton, ein 1,2-Diketon wie Diacetyl oder Pentandion-2,3 ein β-Aminoalkohol wie 2-Aminoäthanol, 2-Aminophenol, 2-Amino-1-butanol und Ephedrin, ein β-Aminoaldehyd, ein β-Aminoketon, ein 1,2-Diimid, ein β-Aminoäther wie 2-(Aminomethyl)tetrahydrofuran, ein aromatisches N-Oxid wie 2,2'-Bipyridin-N,N-dioxid und Pyridin-N-oxid, ein 1,2-Diamin wie Äthylendiamin oder eine Hydroxycarbonsäure wie Weinsäure und ihre Ester.

Der an das Rheniumatom koordinierte Ligand L kann auch an weitere Metalle koordinativ gebunden sein, z.B. in $CH_3ReO_3 \cdot [(NH_2C_6H_5)Cr(CO)_3]$, $CH_3ReO_3 \cdot [(NC_4H_4)Mn(CO)_3]$ und $CH_3ReO_3 \cdot$ [Aminoferrocen].

Die Verbindungen der Formel III lassen sich aus einfache Weise erhalten, indem man die Vorstufe $R^{11}ReO_3$, z.B. $CH_3ReO_3$, in einem organischen Lösungsmittel, z.B. Methylenchlorid, Diäthyläther, Tetrahydrofuran oder Toluol, mit dem entsprechenden Liganden L bei Raumtemperatur umsetzt und dann die Lösung unter vermindertem Druck zur Trockne bringt bzw. das Addukt durch Kristallisation gewinnt. Bei Bedarf kann das Produkt destillativ, durch Umkristallisation oder durch Sublimation unter vermindertem Druck gereinigt werden. Die resultierenden Substanzen sind in der Regel gegen Luft, Temperatur und Wasser stabil.

Als flüssiges Medium für die Ausführung der Oxidationsreaktionen eignen sich organische Lösungsmittel, z.B. Tetrahydrofuran, einwertige Alkohole mit 1 bis 5 C-Atomen, wie Methanol, Äthanol, die verschiedenen Propanole und Butanole, und aromatische Kohlenwasserstoffe wie Toluol und die verschiedenen Xylole. Als flüssiges Medium eignet sich auch ein Zweiphasen-System von Wasser einerseits und mit Wasser nicht mischbaren organischen Lösungsmitteln andererseits, in dem das Wasserstoffperoxid gelöst ist. Insbesondere eignet sich eine Lösung von Wasserstoffperoxid in tert.Butanol.

In der einfachsten Ausführungs- und Anwendungsform der Erfindung geht man folgendermaßen vor :

a) Es wird eine Katalystor-Lösung bereitet, enthaltend ca. 10-30 mg $CH_3ReO_3$. Wird für die Bereitung dieser Lösung tert.-Butanol verwendet, so ist diese tiefgelb gefärbt und bei $-30°$ C, z.B. in einer Kühltruhe, wochenlang lagerfähig, ohne daß die Aktivität nachläßt. Dies ist ein besonderer Vorzug für die erfindungsgemäßen Katalysatoren. Die Lösung muß also nicht für jeden Ansatz frisch zubereitet werden. Eine Vergrößerung der Ansätze auf ein Vielfaches ist möglich, ohne daß Nachteile eintreten.

b) Die unter a) hergestellte Lösung wird unter Rühren dem zu oxidierenden Kohlenwasserstoff, der evtl. in einem nichtoxidierbaren organischen Lösungsmittel gelöst ist, so zugegeben, daß die stark exotherme Oxidationsreaktion nicht zum Sieden der Mischung führt. Für die Reaktionsführung gibt es dann verschiedene Möglichkeiten. Zweckmäßig arbeitet man im Temperaturbereich von 0°C (Eiskühlung) bis 50°C. Jedoch kann man auch bei tieferen Temperaturen arbeiten. Wenn man bei verhältnismäßig niedrigen Temperaturen, z.B. $-30$ bis $+10°C$ arbeitet, verläuft die Reaktion zwar mit sinkender Temperatur immer langsamer, jedoch bildet sich bei der Oxidation der Olefine immer selektiver das Epoxid, und die Weiterreaktion zum Diol wird mit sinkender Temperatur unterdrückt. Strebt man von vornherein die Bildung der 1,2-Diole an, empfiehlt es sich, bei Temperaturen über 10°C in dem angegebenen Bereich zu arbeiten. Die Bildung der 1,2-Diole wird dann dadurch begünstigt, daß die Oxidation stark exotherm ist und das Reaktionsmedium sich um so mehr erhitzt, je höher die Anfangstemperatur ist und infolgedessen je schneller die Reaktion einsetzt.

Sowohl bei der Oxidation der Olefine als auch der Cumulene und Alkine enthält das Reaktionsmedium zweckmäßig 1 bis 30 Gew.-% $H_2O_2$. Wenn besondere Sicherheitsmaßnahmen angewandt werden, können auch Lösungen verwendet werden, in denen der $H_2O_2$-Anteil höher ist. Zweckmäßig sollte die Lösung kein Wasser enthalten, wenngleich ein Wassergehalt nicht schädlich ist, da sich bei der Reaktion aus dem Wasserstoffperoxid in stöchiometrischer Menge Wasser bildet.

c) Falls überschüssiges Peroxid vorhanden ist, kann dieses durch Zusatz von $MnO_2$ katalytisch zerstört werden. Dann wird der Ansatz nach üblichen Methoden, z.B. destillativ, aufgearbeitet. Der Katalysator $CH_3ReO_3$ kann zurückgewonnen werden (Nachweis durch eine Kombination von Gaschromatographie, Massen-Spektrometrie und Infrarot-Spektroskopie).

Die erfindungsgemäß verwendeten Katalysatoren zeichnen sich durch eine hohe Leistungsfähigkeit aus. Der Katalysator ist in dem Reaktionsmedium in allgemeinen in einer Menge von 0,01 bis 7 Gew.-%, vorzugsweise von 0,03 bis 1,0 Gew.-% enthalten. Jedoch kann er in einzelnen Fällen auch in größeren oder kleineren Konzentrationen angewandt werden. Ein Verbrauch findet während der Reaktion, wie es dem Begriff des Katalysators entspricht, praktisch nicht statt. Es ist ohne weiteres möglich, beispielsweise die zehntausendfache Molmenge Olefin mit dem Katalysator zu oxidieren, ehe eine Regenerierung erforderlich ist. Natürlich hängt die Geschwindigkeit der Oxidation auch von der Reaktionstemperatur ab.

Oxidiert man Olefine der Formel II mit Hilfe der erfindungsgemäß vorgeschlagenen Katalysatoren, so werden diese im Temperaturbereich von −30 bis +40°C zunächst selektiv in die entsprechenden Epoxide übergeführt, wie sich aus einer kinetischen Studie an den Beispielen Cyclohexen sowie trans-4-Octen ergibt. Die Selektivität ist vielfach um so ausgeprägter, je konstanter die Temepratur gehalten wird. Auch der Umsetzungsgrad wird durch konstante Temperaturführung vielfach günstig beeinflußt. Die optimalen Bedingungen richten sich im Einzelfall häufig auch nach der Konstitution des zu oxidierenden Alkens oder Alkins. In einer Folgereaktion können aus zunächst gebildeten Epoxiden die entsprechenden 1,2-Diole enstehen. Bei den mit $OsO_4$ katalysierten Olefin-Oxidationen nach Milas et al., l.c., entstehen, außer bei Allylalkoholen, immer die 1,2-Diole und Folgeoxidationsprodukte wie Ketole, nicht aber Epoxide.

Nach einer weiteren Ausgestaltung der Erfindung kann der Katalysator $CH_3ReO_3$ auch in der Form angewendet werden, daß er auf ein Polymeres, das Amin- oder Amid-Stickstoff enthält, aufgezogen (immobilisiert) ist. Dabei bildet sich im allgemeinen eine Verbindung $(Polymer)_f \cdot (CH_3ReO_3)_g$ (VI), in der die Rheniumverbindung über Amin- oder Amid-Stickstoff gebunden ist und in der der Quotient g/f das Gewichtsverhältnis der beiden Bestandteile wiedergibt und im Bereich zwischen 0,01 und 0,2, vorzugsweise zwischen 0,02 und 0,1 liegt. Die einzusetzenden Polymeren besitzen also an ihrer Oberfläche stickstofftragende Gruppen, wobei das Stickstoffatom sowohl in aromatische als auch in aliphatische und darüberhinaus in Amidgruppen eingebaut sein kann (z.B. Poly(4-vinylpyridin) ; das unter der Bezeichnung Reillex vertrieben wird — s. Aldrich-Katalog Nr. 22,696-3). Die Polymersubstanz muß dabei nicht nur als Träger dienen, sondern kann über die Wahl geeigneter Substituenten auch als reaktionssteuernde Komponente (Promotor) wirken. Über den Belegungsgrad des Trägermaterials kann darüberhinaus die Reaktionsgeschwindigkeit, der Umsatz und die Selektivität der Oxidation gesteuert werden. Der große Vorteil der Verbindungen VI besteht darüberhinaus auch darin, daß der Katalysator nach erfolgter Reaktion leicht, z.B. durch einfaches Abfiltrieren, wieder abgetrennt und regeneriert werden kann, wobei die Aktivität, aber auch Umsatz und Selektivität, in nachfolgenden Reaktionsansätzen, wenn überhaupt, nur geringfügig gemindert sind.

Nach einer vorteilhaften Ausführungsform kann man auch so vorgehen, daß ein gelöster Katalysator der Formel I, worin $R^1CH_3$ ist, verwendet wird und dieser nach erfolgter Verwendung durch Umsetzung mit einem Polymeren, das Amin- bzw. Amid-Stickstoff enthält, aus der Lösung entfernt und entweder als Verbindung der Formel VI erhalten wird oder aus dieser Form durch Behandlung im Hochvakuum bei einer Temperatur von mindestens 200°C wieder als Verbindung der Formel I, worin $R^1CH_3$ ist, regeneriert wird.

In den Charakterisierungen einzelner Stoffe in den folgenden Beispielen bedeutet : s Singulett, d Dublett, t Triplett, q Quartett, sept Septett, m Multiplett, br breite Signalform, sst sehr stark, st stark und EI-MS Electron-Impact-Massenspektrum.

Für die nachstehenden Beispiele wurden die Ausgangsstoffe wie folgt hergestellt :

A) Herstellung von Methylrheniumtrioxid $CH_3ReO_3$

Die Synthese von $CH_3ReO_3$ aus $Re_2O_7$ und $Sn(CH_3)_4$ verläuft nur dann erfolgreich, wenn unter strengem Feuchtigkeitsausschluß gearbeitet wird. Es sind deshalb gut getrocknete Lösungsmittel erforderlich. Zweckmäßig wird das Reaktionsgefäß (z.B. Schlenkrohr) vor Verwendung einige Zeit bei 400-600°C im Hochvakuum gehalten, bevor das $Re_2O_7$ eingewogen wird.

10,00 g (20,64 mmol) Dirheniumheptoxid $Re_2O_7$ (76,8 Gew.-% Re) wurden in 90 ml wasserfreiem Tetrahydrofuran gelöst. Dabei wurde das Lösungsmittel unter kräftigem Rühren, z.B. mittels eines Magnetrührwerks, rasch zugegeben, um ein Zusammenbacken von $Re_2O_7$ zu vermeiden. Zu der so erhaltenen Lösung, die leicht bräunlich gefärbt sein kann, gab man 3,15 ml (22,71 mmol ; 1,1-facher Überschuß) handelsübliches Tetramethylstannan $Sn(CH_3)_4$. Da dieses Reagens toxisch ist, sind alle Arbeiten unter geeigneten Vorsichtmaßnahmen, z.B. in einem gut ziehenden Abzug auszuführen. Nun wurde das Reaktionsgemisch 4 Stunden unter Rückfluß gekocht. Längere Reaktionszeiten beeinflußten die Reaktion nicht negativ. Nach Abkühlen der Lösung auf Raumtemperatur wurde das Lösungsmittel langsam im Vakuum einer Ölpumpe entfernt, bis der Rückstand eine breiige Konsistenz annahm. Danach können merkliche Mengen $CH_3ReO_3$ absublimieren. Das weitere Entfernen des Lösungsmittels erfolgte deshalb erst, nachdem die Apparatur mit einem Kühlfinger versehen war, der auf Temperaturen zwischen $-10°C$ und $0°C$ gekühlt war (Umwälzkryostat).

Nachdem das Lösungsmittel restlos entfernt war, wurde das Produkt bei etwa 80°C bei dem genannten Vakuum durch Sublimation auf den gekühlten Sublimationsfinger in farblosen Nadeln gewonnen. Die Ausbeute an analysenreiner Substanz betrug 4,85 g (94% der Theorie, gemäß der Reaktionsgleichung $Re_2O_7$ + $Sn(CH_3)_4 \rightarrow CH_3ReO_3 + [(CH_3)_3SnO]ReO_3$).

Der graue Rückstand wurde in 40 ml Tetrahydrofuran aufgenommen. Die Lösung wurde über eine ca. 2 cm hohe $Al_2O_3$-Schicht filtriert. Aus dem farblosen Filtrat konnte durch Entfernen des Lösungsmittels die Verbindung $[(CH_3)_3SnO]ReO_3$ als farbloses Pulver analysenrein gewonnen werden ; die Ausbeute betrug 8,12 g (95% der Theorie bezüglich der oben angegebenen Gleichung).

**Daten für Methylrheniumtrioxid**

Schmelzpunkt 106°C. — IR ($cm^{-1}$, KBr) : 1002 sst, 950 sst, br [v(Re = 0)]. — $^1$H-NMR ($CDCl_3$, 28°C) : $\delta(CH_3)$ = 2,61 s. — $^{13}$C-NMR ($CDCl_3$, 28°C) : $\delta(CH_3)$ = 19,03 [$^1$J(C,H) = 138 Hz]. — $^{17}$O-NMR ($CDCl_3$, 28°C) : $\delta(O)$ = 829 ppm. — EI-MS : m/z — 248/250 (Molekül-Ion, mit korrektem Isotopenmuster $^{185}$Re/$^{187}$Re). — Die Substanz kann bei Raumtemperatur unzersetzt aufbewahrt werden. —

$CH_3ReO_3$ : Ber. : C 4,82, H 1,20, O 19,26, Re 74,72 :
(249,21) : Gef. : C 4,84, H 1,19, O 19,30, Re 74,78.

B) Herstellung der Katalysatorlösung

100 ml tert.-Butanol wurden mit 25 ml 30%igem $H_2O_2$ (pro analysi-Qualität) versetzt und mit 30 g wasserfreiem $MgSO_4$ eine Stunde lang gerührt. Das Magnesiumsulfat wurde dann abfiltriert. Die erhaltene Oxidationslösung wurde vorsorglich unter Stickstoffatmosphäre aufbewahrt. Zu 2,68 ml dieser Lösung wurden 20 mg festes Methylrheniumtrioxid gegeben. Die Lösung, bestehend aus dem Katalysator $CH_3ReO_3$ (30 mmol/Liter) und dem Oxidationsmittel $H_2O_2$, verfärbte sich augenblicklich gelb. Sie ist bei 0°C längere Zeit haltbar und behält bei dieser und noch niedrigerer Temperatur ihre Aktivität. Die Katalysatormenge kann auf 3 mmol/Liter reduziert werden, doch nimmt dann die Oxidationsreaktion bei gleichem Umsatz einen längeren Zeitraum in Anspruch.

**Beispiele**

**Beispiele 1-35 — Oxidation von Alkenen, Cumulenen und Alkinen**

Die nach B hergestellte Katalysatorlösung wurde mit dem betreffenden Alken oder Alkin (s. Tabelle 1) versetzt, wobei eine starke Erwärmung des Ansatzes, manchmal bis zur Siedehitze, eintrat. Die Menge des eingesetzten Alkens und Cumulens entsprach bezüglich der Zahl der Doppelbindungen der Molmenge des vorhandenen $H_2O_2$. Bei der Oxidation von Alkinen wurde je Dreifachbindung die doppelte Menge $H_2O_2$ verwendet. Nach 5 bis 30 Minuten hatte sich die Lösung wieder entfärbt, und die Reaktion war zum Stillstand gekommen, d.h. das eingestellte Gleichgewicht änderte sich auch nach längerer Zeit nicht mehr.

Um das in der Lösung noch vorhandene überschüssige $H_2O_2$ zu entfernen, löste man den ganzen Ansatz in 10 ml Tetrahydrofuran und gab zur Zersetzung des Peroxids 1 g Braunstein $MnO_2$, d.i. einen Überschuß zu. Nach 10 min war die Gasentwicklung beendet. Man filtrierte die Suspension über eine mit ®Celite (eingetragenes Warenzeichen der Fa. Manville Corp., Denver, USA) beschickte Glasfritte und wusch dreimal mit Tetrahydrofuran nach. Das Lösungsmittel wurde vom Filtrat unter vermindertem Druck entfernt. Das so erhaltene Rohprodukt war nach dreimaligem Waschen mit je 20 ml n-Hexan analysenrein. Falls notwendig, wurde das Produkt durch Destillation order Umkristallisieren, gegebenenfalls bei erniedrigter Temperatur, aufgearbeitet.

Durch Anwendung eines Überschusses an $H_2O_2$ läßt sich die Ausbeute in vielen Fällen deutlich verbessern.

EP 0 380 085 B1

## Tabelle 1 für Oxidationsreaktionen

| Beispiel/Ausgangs-material | Reaktions-bedingungen* | Produkte | Ausbeute |
|---|---|---|---|
| 1 Propylen | -10°C, 12 h | Propylenoxid<br>1,2-Propandiol | 50 % **<br>50 % |
| 2 2-Butylen | -10°C, 6 h | 2,3-Butandiol | 100 % ** |
| 3 1-Penten | RT, 1 h | 1,2-Epoxypentan | 55 % |
| 4 cis-2-Penten | RT, 3 h | Pentan-2,3-diol<br>2,3-Epoxypentan | 10 %<br>90 % |
| 5 2-Methyl-1-buten | RT, 4 H | Epoxy-2-methyl-butan | 65 % |
| 6 2,3-Dimethyl-2-buten | RT, 1 h | 2,3-Dimethyl-2,3-butandiol | 75 % |
| 7 2-Methyl-1-hexen | RT, 3 h | 1,2-Epoxy-2-methylhexan | 55 % |
| 8 1-Octen | RT, 3 h | 1,2-Octandiol | 70 % |
| 9 trans-4-Octen | RT, 2 h | 4,5-Epoxyoctan | 100 % |
| 10 1-Decen | RT, 3 h | 1,2-Epoxydecan | 60 % |
| 11 Cyclohexen | RT, 1 h | Cyclohexan-1,2-diol (trans-Isomeres) | 98 % |
| 12 1-Methyl-1-cyclohexen | RT, 2 h | 1,2-Dihydroxy-1-methyl-cyclohexan | 70 % |
| 13 Cyclohepten | RT, 1 h | Epoxycycloheptan | 80 % |
| 14 Cycloocten | RT, 1 h | Epoxycyclooctan | 80 % |
| 15 cis,cis-1,5-Cyclo-octadien | RT, 2 h | 1,2,5,6-Diepoxy-cyclooctan | 80 % |
| 16 Cyclooctatetraen | RT, 2 h | 1,2-Epoxy-3,5,7-cyclooctatrien | 35 % |
| 17 Cyclododecen | RT, 1 h | Epoxycyclodo-decan | 100 % |
| 18 1,9-Cyclohexa-decadien | RT, 3 h | 10-Epoxy-1-cyclohexadecen | 55 % |
| 19 α-Pinen | - 30°C, 1 d | exo- und endo-Epoxypinan | 55 % |

8

| Beispiel/Ausgangs-material | Reaktions-bedingungen* | Produkte | Ausbeute |
|---|---|---|---|
| 20 Limonen | RT, 5 h | Limonen-mono-epoxid | 35 % |
| 21 Citral | RT, 1 d | 2,3-Epoxycitral | 28 % |
| 22 Squalen | RT, 2 h | Dihydroxysqualen | 75 % |
| 23 Allylalkohol | RT, 10 h | 2,3-Epoxy-1-propanol | 90 % |
| 24 2-Brom-2-buten | RT, 2 h | 2,3-Epoxy-2-brombutan | 45 % |
| 25 Maleinsäure-diäthylester | RT, 1 d | Epoxybernstein-säure-diäthyl-ester | 20 % |
| 26 Ölsäure-methyl-ester | RT, 1 d | 9,10-Dihydroxy-octadecansäure-methylester | 92 % |
| 27 Linolsäure-methylester | RT, 2 h | 6,7-Epoxylinol-säuremethylester | 80 |
| 28 1,4-Naphthochinon | 70°C, 18 h | 2,3-Epoxy-1,4-naphthochinon | 64 % |
| 29 3-Methyl-1,2-butadien | RT, 1 h | 3-Hydroxy-3-methyl-2-butanon | 45 % |
| 30 3-Hexin | RT, 1 h | 4-Hexen-3-on | 40 % |
| 31 Diphenylacetylen | 65°C, 1 d | Benzil | 71 % |
| 32 Styrol | RT, 2 h | Phenylacetaldehyd<br>(±)-Phenyläthylen-glykol<br>Benzaldehyd | 12 %<br>11 %<br>25 % |
| 33a) cis-Stilben (1,2-Diphenyläthylen) | 70°C, 2 h | Benzaldehyd<br>cis-Stilbenoxid | 76 %<br>6 % |
| b) cis-Stilben (1,2-Diphenyläthylen) | RT, 2 h | Benzaldehyd<br>cis-Stilbenoxid | 49 %<br>1 % |
| 34) 2,3,4,5,6-Penta-fluorstyrol | RT, 3 h | 2,3,4,5,6,-Penta-fluorstyroloxid | 56 % |

\* RT = Raumtemperatur (ca. 28° C), h = Stunde, d = Tag
\*\* Ausbeute auf umgesetztes Olefin bezogen.

**35)**

50 mg (0,2 mmol) Methylrheniumtrioxid wurden unter Rühren bei −10°C in 135 ml Oxidationslösung nach B gelöst. Bei dieser Temperatur wurden 25 ml Cyclohexen (20 g, 0,24 mol) über einen Zeitraum von 3 Stunden zugetropft. Die Farbe der Lösung hellte dabei etwas auf. Nachdem das Cyclohexen vollständig zugegeben war, ließ man auf Raumtemperatur kommen. Überschüssiges Wasserstoffperoxid wurde durch Rühren mit 2 g handelsüblichem $MnO_2$ innerhalb von 1 Stunde zerstört. Die Suspension wurde über eine D3-Fritte/Celite abfiltriert, dreimal mit je 20 ml Tetrahydrofuran nachgewaschen und das Lösungsmittel im Vakuum einer Ölrotationspumpe abgezogen. Der klebrige Rückstand wurde fünfmal mit je 50 ml n-Hexan gewaschen und dann im Hochvakuum getrocknet. Ausbeute 24,1 g farbloses, pulvriges 1,2-trans-Cyclohexandiol (85%). Katalysator/Produkt-Molverhältnis — 1/1000.

**36) Oxidation mit einer Verbindung mit 6-wertigem Rhenium**

Von der nach B) aus tert.-Butanol und $H_2O_2$ zunächst bereiteten "Oxidationslösung" wurde ein Anteil von 5 ml mit 20 mg festem Tetramethyltetraoxodirhenium (VI) $(CH_3)_4Re_2O_4$ versetzt. Dabei ergab sich augenblicklich eine Gelbfärbung. Es wurden dann 0,15 ml Cyclododecen zugegeben. Dabei erwärmte sich die Lösung geringfügig. Nach 1 Stunde wurde wie bei den Beispielen 1-34 aufgearbeitet und analysiert (GC/IR/MS). Dabei erhielt man 99% Epoxycylcododecan.

**Verbindungen der Formel III — Beispiele 37-52**

37) (1-Azabicyclo[2,2,2]octan)methyl(trioxo)rhenium (VII), $CH_3ReO_3 \cdot N(C_2H_4)_3CH$

Zu einer Lösung von 70 mg (0,63 mmol) Chinuclidin [$N(C_2H_4)_3CH$] in 10 ml Tetrahydrofuran wurden bei 20°C unter ständigem Rühren 160 mg (0,64 mmol) $CH_3ReO_3$ gegeben. Die zunächst farblose Lösung färbte sich bei der Zugabe des Chinuclidins schlagartig zitronengelb. Nach einer Stunde wurde das Lösungsmittel unter vermindertem Druck entfernt. Auf diese Weise wurden 210 mg (91%) des Produktes in Form kleiner kristallnadeln erhalten. Durch Umkristallisieren aus Tetrahydrofuran/n-Pentan (Volumenverhältnis 3 : 1) können größere Kristalle gezüchtet werden. Fp. 112°C (Zers.).

| | |
|---|---|
| $C_8H_{16}NO_3Re$ | Ber. : C 26,65, H 4,44, N 3,89, |
| (360, 43) | Gef. : C 27,08, H 4,37, N 3,79 |

$^{17}O$-NMR-Spektrum : $\delta \underline{O}$ = 566 ppm [$CH_2Cl_2/CDCl_3$]. Die Verbindung ist durch Einkristall-Röntgen-Strukturanalyse charakterisiert : d(Re-O) = 165-173 pm, d(Re-N) = 250 pm
IR [KBr] : v(Re = 0), cm$^{-1}$ : 925 sst.
$^1$H-NMR [Solvens] $\delta$(ppm, 23°C) : 1,40 (s, 3H) ; 1,51 (dt, $^3J(H,H)$ = 8,06 Hz, $^3J(H,H)$ = 3,18 Hz, 6H) ; 1,81 (sept, $^3J(H,H)$ = 3,18 Hz, 1H) ; 2,48 (t, $^3J(H,H)$ = 8,06 Hz, 6H) [$CDCl_3$]

38) (1,4-Diazabicyclo[2,2,2]octan)-N,N'-bis[methyl(trioxo)rhenium (VII)], $[CH_3ReO_3]_2 \cdot [N(C_2H_4)_3N]$

Zu einer Lösung von 130 mg (0,52 mmol) $CH_3ReO_3$ in 10 ml Tetrahydrofuran wurden 58 mg (0,52 mmol) 1,4-Diazabicyclo[2,2,2]-octan gegeben. Die Lösung färbte sich dabei sofort intensiv gelb. Nach 1 Stunde wurde das Lösungsmittel unter vermindertem Druck entfernt und das gelbe, feinkristalline Produkt bei −30°C aus Tetrahydrofuryan umkristallisiert. Die Ausbeute an analysereinem Produkt betrug 160 mg (85%).

| | |
|---|---|
| $C_8H_{16}N_2O_6Re_2$ | Ber : C 15,73, H 2,95, H 2,95, N 4,59, O 15,72 ; |
| (610,65) | Gef : C 15,78, H 2,95, N 4,47, O 15,74. |

IR [KBr] : v(Re = 0), cm$^{-1}$ : 909 sst
$^1$H-NMR [Solvens] $\delta$(ppm, 23°C) : 1,47 (s, 6H) ; 2,55 (s, 12H) [$CDCl_3$]

39) (Anilin)methyl(trioxo)rhenium(VII), $CH_3ReO_3 \cdot NH_2C_6H_5$

Eine Lösung von 100 mg (0,40 mmol) $CH_3ReO_3$ in 10 ml Toluol wurde mit 36 µl (37 mg, 0,40 mmol) frisch destilliertem Anilin versetzt. Dabei schlug die Farbe augenblicklich nach gelb um. Nach 15 min wurde das Lösungsmittel unter vermindertem Druck entfernt und der Rückstand bei 40°C (1,33 · 10$^{-3}$ mbar) sublimiert. Auf diese Weise erhielt man 120 mg (88%) des Produktes als hellgelbe Kristalle.

| | |
|---|---|
| $C_7H_{10}NO_3Re$ | Ber. : C 24,56, H 2,94, N 4,09, O 14,02, Re 54,39 ; |
| (342,37) | Gef. : C 24,49, H 2,99, N 4,09, O 13,81, Re 54,62. |

**10**

$^{17}$O-NMR-Spektrum : $\delta\underline{O}$ = 566 ppm [CH$_2$Cl$_2$/CDCl$_3$].
— Die Verbindung ist ferner durch Einkristall —
Röntgenstrukturanalyse charakterisiert : d(Re-O) = 169,9 pm ; d(Re-N) = 246,9 pm ; es liegen NH$_2$... O- Wasserstoffbrückenbindungen vor.
IR [KBr] : v(Re = 0), cm$^{-1}$ : 927 sst.
$\underline{^1}$H-NMR [Solvens] δ (ppm, 23°C) : 2,57 (s, 3H) ; 3,62 (br, 2H) ; 6,67 (dd, $^3$J(H,H) = 7,32 Hz, $^4$J(H,H) = 1,22 Hz, 2H) ; 6,75 (t, $^3$J(H,H) = 7,32 Hz, 1H) ; 7,14 (m ; 2H) [CDCl$_3$]

40) (2,2′-Bipyridin)methyl(trioxo)rhenium (VII), CH$_3$ReO$_3$ · C$_{10}$H$_8$N$_2$

Eine Lösung von 1,00 g (4,0 mmol) CH$_3$ReO$_3$ in 100 ml Toluol wurde bei Raumtemperatur unter ständigem kräftigem Rühren mit einem geringen Überschuß (0,79 g, 4,4 mmol) an 2,2′-Bipyridin versetzt. Es fiel sofort ein gelber Niederschlag aus. Nach einer Reaktionszeit von 30 min ließ man den Niederschlag absitzen und dekantierte das Lösungsmittel ab. Um überschüssiges 2,2′-Bipyridin zu entfernen, wusch man noch dreimal mit jeweils 25 ml Toluol nach. Dieser Vorgang wurde noch zweimal mit n-Pentan wiederholt. Das Lösungsmittel wurde schließlich im Vakuum einer Ölpumpe entfernt. Auf diese Weise wurden 1,59 g (97%) der Verbindung analysenrein erhalten. Fp. 138°C (Zers.).

C$_{11}$H$_{11}$N$_2$O$_3$Re      Ber. : C 32,56, H 2,71, N 6,91, O 11,84 ;
(405,43)      Gef. : C 32,68, H 2,74, N 6,80, O 12,02

IR [KBr] : v(Re = 0), cm$^{-1}$ : 910 sst, 940 sst
$\underline{^1}$H-NMR [Solvens] δ(ppm, 23°C) : 1,95 (s, 3H) ; 7,35 (m, 2H) ; 7,89 (m, 2H) ; 8,50 (d, $^3$J(H,H) = 7,93 Hz, 2H) ; 8,66 (d, $^3$J(H,H) = 4,88 Hz, 2H) [CD$_2$Cl$_2$]

41) (2,2′-Bipyridin)methyl(oxo)dichlororhenium(V), CH$_3$ReOCl$_2$ · C$_{10}$H$_8$N$_2$

Zu 0,50 g (1,23 mmol) einer Suspension der Verbindung nach Beispiel 30 in 30 ml Methylenchlorid tropfte man bei Raumtemperatur unter ständigem Rühren 314 μl (2,44 mmol) frisch destilliertes Trimethylchlorsilan. Nun gab man die äquimolare Menge (322 mg ; 1,23 mmol) Triphenylphosphan zu. Sofort färbte sich die Lösung tiefviolett. Nun wurde das Reaktionsgemisch so lange unter Rückfluß gekocht, bis die violette Farbe vollständig verschwunden und die Reaktionslösung gelblich-braun gefärbt war (Reaktionszeit etwa 1 Stunde). Nach dem Abkühlen der Lösung auf Raumtemperatur wurde das Produkt durch Zugabe von 60 ml n-Pentan quantitativ ausgefällt und das Lösungsmittel, das nur noch schwach rot gefärbt war, abdekantiert. Durch mehrmaliges Waschen mit jeweils 10 ml Toluol wurde das Produkt gereinigt. Durch Umkristallisieren aus Methylenchlorid bei −30 °C konnten kleine, quaderförmige Kristalle gezüchtet werden. Man erhielt die Verbindung in 98%iger Ausbeute (0,54 g).

C$_{11}$Cl$_2$H$_{11}$N$_2$ORe (444,33)
Ber. : C 29,73, H 2,50, N 6,30, O 3,60, Cl 15,96 ;
Gef. : C 29,64, H 2,51, N 6,20, O 3,63, Cl 16,01

IR [KBr] : v(Re = 0), cm$^{-1}$ : 988 sst
$\underline{^1}$H-NMR[Solvens] δ(ppm, 23°C) : 5,41 (s, 3H) ; 7,09 (m, 1H) ; 7,46 (m, 1H) ; 7,63 (m, 1H) ; 7,90 (m, 1H) ; 8,00 (m, 2H) ; 8,36 (m, 1H) ; 8,47 (m, 1H) [CD$_2$Cl$_2$]

42) (2,2′-Bipyridin)methyl(oxo)bis(trimethylsilyl-methyl)rhenium(V), CH$_3$ReO[CH$_2$Si(CH$_3$)$_2$ · C$_{10}$H$_8$N$_2$

Eine Lösung von 1,0 ml (Trimethylsilyl)methylmagnesiumchlorid, (CH$_3$)$_3$SiCH$_2$MgCl (1,0 molare Lösung in Diäthyläther ; 1,0 mmol), wurde mit 10 ml Toluol verdünnt, dann auf −78°C gekühlt und schließlich mit einer ebenfalls −78°C kalten Suspension von 175 mg (0,39 mmol) der Verbindung nach Beispiel 41 in 20 ml Toluol versetzt. Nun ließ man das Reaktionsgemisch innerhalb von 2 Stunden auf Raumtemperatur erwärmen. Oberhalb von etwa −30°C war eine leichte Grünfärbung zu erkennen ; schließlich war eine blaugrüne Lösung entstanden. Nachdem sich die Lösung auf Raumtemperatur erwärmt hatte, rührte man noch 1 Stunde und entfernte schließlich das Lösungmittel im Vakuum einer Ölpumpe. Der Rückstand wurde auf eine Chromatographiesäule aufgebracht und bei 10°C an Kieselgel (Fa. Merck ; Kieselgel 60, Korngröße 0,063-0,200 mm) chromatographiert. Das Produkt wurde als tiefgrüne Zone mit n-Pentan/Toluol (Volumenverhältnis 1 : 1) eluiert.

11

Dieses Lösungsmittelgemisch ist für die Züchtung von Einkristallen geeignet. Aus dem Eluat wurden beim Eindampfen im Vakuum einer Ölpumpe 60 mg (28%) der gegen Luft und Wasser unempfindlichen Verbindung erhalten.

$C_{19}H_{33}N_2ORe Si_2$     Ber. : C 41,65, H 6,07, N 5, 11 ;
(547,86)            Gef. : C 41,72, H 6,36, N 4,42.

IR (KBr) : v(Re = 0), cm$^{-1}$ : 975 sst
1H-NMR [Solvens] δ(ppm, 23°C) : 0,34 (s, 18H) ; 2,22 (d, $^2J(H,H)$ = 10,98 Hz, 2H) ; 3,15 (d, $^2J(H,H)$ = 10, 98 Hz, 2H) ; 3,67 (s,3H) ; 5,89, 6,26, 6,35, 6,58, 6,69, 7,19, 7,71, 8,72 (m, 8H) [d$^8$-Toluol]

43) (2,2′-Bipyridin)trimethyl(oxo)rhenium, $(CH_3)_3ReO \cdot C_{10}H_8N_2$

Zu einer Suspension von 888 mg (2,0 mmol) der Verbindung nach Beispiel 41 in 60 ml Toluol tropfte man bei −78°C unter ständigem Rühren 2,69 ml Methyllithium (1,6 molare Lösung in Diäthyläther ; 4,3 mmol). Die Reaktionslösung wurde etwa 30 min bei dieser Temperatur gerührt und anschließend innerhalb von 3 Stunden auf Raumtemperatur gebracht. Bereits bei einer Temperatur von −65°C kam es zur Reaktion (Grünfärbung des Reaktionsgemisches). Nach 3 Stunden hatte die intensiv grün gefärbte Lösung Raumtemperatur erreicht, und es war das Ausfallen eines feinen Niederschlages an LiCl zu beobachten. Nun wurde das Lösungsmittel unter vermindertem Druck entfernt und der dunkle, fast schwarze Rückstand durch Aufnehmen in Toluol extrahiert. Die tiefgrüne Lösung wurde über ®Celite filtriert, um das ausgefallene LiCl gänzlich zu entfernen, und das Lösungsmittel dann unter vermindertem Druck abgezogen. Man erhielt 726 mg (90%) der Verbindung als fast schwarzes, an der Luft stabiles Pulver, das sich in n-Pentan und Toluol mit tiefgrüner Farbe, in Diäthyläther, Tetrahydrofuran und Aceton tiefblau und in Methylenchlorid und Chloroform türkisfarben löste.

$C_{13}H_{17}N_2ORe$     Ber. : C 38,67, H 4,24, N 6,94, O 3,97
(403,40)           Gef. : C 38,70, H 4,10, N 6,79, O 4,00

IR [KBr] : v(Re = 0), cm$^{-1}$ : 976 sst
1H-NMR [Solvens] δ(ppm, 23°C) : 2,40 (s, 6H) ; 3,47 (s, 3H) ; 6,84, 7,19, 7,57, 7,86, 7,91, 8,28, 8,79 (m, 8H) [CDCl$_3$]

44-46) Isomere von (Methoxyanilin)methyl(trioxo)rhenium(VII), $CH_3ReO_3 \cdot CH_3OC_6H_4NH_2$

Zu einer Lösung von 80 mg (0,32 mmol) CH$_3$ReO$_3$ in 10 ml Toluol wurden 40 mg (0,33 mmol) des entsprechend substituierten Anilin-Derivates gegeben, wobei sich die Lösung spontan gelb färbte. Nach 30 min. wurde das Lösungsmittelvolumen im Vakuum einer Ölpumpe auf die Hälfte reduziert. Dann gab man 10 ml n-Hexan zu. Das jeweilige Produkt (Beispiele 44-46) kristallisierte bei −35°C aus und wurde dann im Hochvakuum getrocknet.

44) 2-Methoxyanilin-Verbindung : Gelbe Kristalle ; Ausbeute 110 mg (92%)

$C_8H_{12}NO_4Re$     Ber : C 25,80, H 3,25, N 3,76 ;
(372,40)          Gef : C 25,75, H 3,15, N 3,76.

IR [KBr] : v(Re = 0), cm$^{-1}$ ; 929 sst
1H-NMR [Solvens] δ(ppm, 23°C) : 2,58 (s, 3H) ; 2,90 (br, 2H) ; 3,83 (s, 3H) ; 6,77 (m, 4H) [CDCl$_3$]

45) 3-Methoxyanilin-Verbindung : Gelbbraune Kristalle ; Ausbeute 110 mg (92%).

$C_8H_{12}NO_4Re$     Ber : C 25,80, H 3,25, N 3,76 ;
(372,40)          Gef : C 25,73, H 3,23, N 3,77.

IR [KBr] : v(Re = 0), cm$^{-1}$ : 915 sst, 944 st, 962 st
1H-NMR [Solvens] δ(ppm, 23°C) : 2,59 (s, 3H) ; 3,75 (s, br, 5H) ; 6,23 (t, $^4J(H,H)$ = 2,44 Hz, 1H) ; 6,27 (dt, $^3J(H,H)$ = 7,93 Hz, $^4J(H,H)$ = 1,22 Hz, 1H) ; 6,31 (dd, $^3J(H,H)$ = 9,16 Hz, $^4J(H,H)$ = 2,44 Hz, 1H) ; 7,04 (t, $^3J(H,H)$ = 7,93 Hz) [CDCl$_3$]

46) 4-Methoxyanilin-Verbindung : Rotbraune Kristalle ; Ausbeute 110 mg (92%).

$C_8H_{12}NO_4Re$      Ber : C 25,80, H 3,25, N 3,76
(372,40)      Gef : C 25,93, H 3,12, N 3,72.

IR [KBr] : v(Re = 0), cm$^{-1}$ : 909 sst, 934 sst, 962 st
1H-NMR [Solvens] δ(ppm, 23°C) : 2,56 (s, 3H) ; 3,39 (br, 2H) ; 3,73 (s, 3H) ; 6,63 (d,$^3$J(H,H) = 9,16 Hz, 2H) ;
6,73 (d, $^3$J(H,H) = 8,13 Hz, 2H) [CDCl$_3$]

47) [2-(Aminomethyl)pryidin]methyl(trioxo)rhenium(VII), $CH_3ReO_3 \cdot NC_5H_4CH_2NH_2$

Eine Lösung von 80 mg (0,32 mmol) CH$_3$ReO$_3$ in 10 ml Toluol wurde mit 33 µl (35 mg, 0,32 mmol) 2-(Aminomethyl)pyridin versetzt. Es bildete sich spontan ein gelber Niederschlag, von dem abdekantiert und der anschließend zweimal mit je 10 ml Toluol gewaschen und zuletzt im Vakuum einer Ölpumpe getrocknet wurde. So erhielt man 110 mg (quantitativ) des gelben Produkts.

$C_7H_{11}N_2O_3Re$      Ber : C 23,53, H 3,10, N 7,84, O 13,43, Re 52,10 ;
(357,38)      Gef : C 23,73, H 3,08, N 7,78, O 13,70, Re 52,05.

IR [KBr] v(Re = 0) cm$^{-1}$ : 908 sst, 935 sst
1H-NMR [Solvens] δ(ppm, 23°C) : 2,12 (s, 3H) ; 3,53 (br, 2H) ; 5,04 (d, $^2$J(H,H) = 22,0 Hz, 1H) ; 6,09 (d, $^2$J(H,H)
= 22,0 Hz, 1H) ; 8,19 (m, 1H) ; 8,30 (m, 1H) ; 8,83 (m, 1H) ; 9,23 (m, 1H) [CD$_3$CN]

48) [N-(2-Aminoäthyl)pyrrolidin]methyl(trioxo)rhenium(VII), $CH_3ReO_3 \cdot (C_4H_8N-CH_2CH_2NH_2)$

Es wurden 41 µl (37 mg, 0,32 mmol) N-(2-Aminoäthyl)pyrrolidin zu einer Lösung von 80 mg CH$_3$ReO$_3$ (0,32 mmol) in 10 ml Toluol gegeben. Es trat sofort eine Gelbfärbung ein. Nach ca. 30 sec. fiel ein gelber Niederschlag aus. Von diesem wurde abdekantiert ; er wurde dann mit 10 ml Toluol gewaschen und das Lösungsmittel im Vakuum einer Ölpumpe entfernt. Man erhielt 100 mg (85%) der Verbindung als gelbes Pulver.

$C_7H_{17}N_2O_3Re$      Ber : C 23,13, H 4,72, N 7,71
(363,43)      Gef : C 23,31, H 4,64, N 7,61

49) [(N,N-Dimethylamino)acetonitril]methyl(trioxo)rhenium(VII), $CH_3ReO_3 \cdot [(CH_3)_2NCH_2CN]$

Eine Lösung von 70 mg (0,28 mmol) CH$_3$ReO$_3$ in einer Mischung aus 5 ml Toluol und 5 ml n-Pentan wurde mit 24 mg (27 µl, 0,28 mmol) (N,N-Dimethylamino)acetonitril versetzt. Aus der leicht gelblichen Lösung fielen beim Abkühlen auf −35°C im Verlaufe von 20-30 h 80 mg (86%) gelblich durchscheinende Kristalle aus, die im Vakuum einer Ölpumpe getrocknet wurden.

$C_5H_{11}N_2O_3Re$      Ber : C 18,02, H 3,33, N 8,40 ;
(333,36)      Gef : C 17,91, H 3,28, N 8,04.

50) [(N,N-Dimethylamino)aceton]methyl(trioxo)rhenium(VII), $CH_3ReO_3 \cdot [(CH_3)_2NCH_2C(O)CH_3]$

Zu einer Lösung von 70 mg (0,28 mmol) CH$_3$ReO$_3$ in 10 ml n-Pentan wurden 28 mg (32 µl, 0.28 mmol) (N,N-Dimethylamino)aceton gegeben. Die anfangs farblose Lösung verfärbte sich sofort gelb. Sie wurde dann für 10-30 h bei −35°C der Kristallisation überlassen. Man erhielt 80 mg (82%) der Verbindung in Form von gelben Plättchen.

$C_6H_{14}NO_4Re$      Ber : C 20,57, H 4,03, N 4,00 ;
(350,39)      Gef : C 20,84, H 4,02, N 3,99.

51) Methyl)pyridin-N-oxid)trioxorhenium(VII), $CH_3ReO_3 \cdot ONC_5H_5$

70 mg (0,28 mmol) CH$_3$ReO$_3$ und 60 mg (0,64 mmol) Pyridin-N-oxid wurden in 10 ml Toluol zusammengegeben. Es bildete sich eine farblose Lösung. Diese wurde bei −35°C aufbewahrt. Nach 10 bis 30 Stunden kristallisierte die Verbindung in gelben Nadeln aus. Das Produkt wurde im Hochvakuum getrocknet. Ausbeute

80 mg (80%).

$C_6H_8NO_4Re$         Ber : C 20,93, H 2,34, N 4,07 ;
(344,34)             Gef : C 21,12, H 2,45, N 4,07.

52) Methyl[tricarbonyl($\eta^5$-pyrrolyl)mangan]trioxorhenium(VII), $CH_3ReO_3 \cdot [(\eta^5-C_4H_4N)]Mn(CO)_3$

Zu einer Lösung von 70 mg (0,34 mmol) Tricarbonyl($\eta^5$-pyrrolyl)mangan ($\eta^5$-$C_4H_4N$)Mn(CO)$_3$ in 10 ml Diäthyläther wurden 85 mg (0,34 mmol) $CH_3ReO_3$ gegeben. Nach 20 Stunden Rühren bei Raumtemperatur wurde filtriert. Dem Filtrat fügte man 10 ml n-Hexan zu und ließ die Lösung 10-30 Stunden bei −35°C kristallisieren. Ausbeute 110 mg (71%) ; gelbbraune Kristalle.

$C_6H_7MnNO_6Re$      Ber : C 21,15, H 1,55, Mn 12,00, N 3,08 ;
(454,29)              Gef : C 21,27, H 1,59, Mn 12,09, N 3,10.

**Beispiele 53-56 — Oxidation mit Verbindungen III**

Zu 2,68 ml der nach B zunächst erhaltenen Oxidationslösung gab man 20 mg $CH_3ReO_3 \cdot$ L. Dabei wurde eine spontane Gelbfärbung beobachtet. Der so eingestellten Katalysatorlösung setzte man 0,5 ml Cyclohexen zu. Im Verlauf von 3 h verschwand die Gelbfärbung und das eingestellte Verhältnis Reaktand/Produkt blieb konstant. Die anschließende Aufarbeitung erfolgte analog der für die Beispiele 1 bis 34 gegebenen Vorschrift. Die Ergebnisse sind aus der folgenden Tabelle 2 ersichtlich.

## Tabelle 2

| Beispiel/Ligand L | | Reaktions-bedingungen | Produkte | Ausbeute |
|---|---|---|---|---|
| 53 | m-Anisidin | RT, 20 h | 1,2-Cyclohexandiol | 50 % |
| 54 | p-Anisidin | RT, 20 h | 1,2-Cyclohexandiol | 55 % |
| 55 | Methylephedrin | RT, 20 h | Epoxycyclohexan | 75 % |
| 56 | Dimethylaminoacetonitril | RT, 20 h | 1,2-Cyclohexandiol | 51 % |

\* RT = Raumtemperatur (ca. 28° C)

**Beispiele 57-63 — Auf einem Polymeren fixierter Oxidationskatalysator**

57) Eine Suspension von 9,20 g Poly(4-vinylpyridin) (Handelsprodukt "Reillex" der Fa. Aldrich Chemie GmbH, Steinheim/Albuch, Art. 22,696-3) in 200 ml Tetrahydrofuran wurde nach Zugabe von 0,80 g (3,2 mmol) $CH_3ReO_3$ 20 Stunden mit einem Magnetrührwerk gut durchmischt. In dieser Zeit verfärbte sich das Polymere unter Bildung einer Komplexverbindung des $CH_3ReO_3$ von farblos nach hellgelb. Das Lösungsmittel wurde dann abdekantiert. Der Rückstand wurde fünfmal mit jeweils 20 ml Tetrahydrofuran gewaschen und zuletzt im Vakuum einer Ölpumpe getrocknet. Es blieb ein gelbes Pulver zurück. Wie sich aus der Elementaranalyse ergibt (Re berechnet 6,0, gefunden 6,1%), war das $CH_3ReO_3$ verlustfrei an das polymere Trägermaterial gebunden. Durch diese Maßnahme wurde die Flüchtigkeit von $CH_3ReO_3$ stark erniedrigt : Die Verbindung ließ sich bis 150° C selbst in einem Hochvakuum nicht mehr vom Trägermaterial entfernen.

Analoge Katalysatoren, in denen $CH_3ReO_3$ auf Polymere aufgezogen war, erhielt man nach derselben Vorschrift mit

14

58) Poly(2-vinylpyridin)                (Aldrich Nr. 18,950-2)
59) Poly(2-vinylpyridin-co-Styrol)      (Aldrich Nr. 18,127-7)
60) Poly(acrylsäureamid)                (Aldrich Nr. 19,207-4)
61) Poly(vinylpyrrolidon)               (Aldrich Nr. 85,648-7)
62) Polyimid                            (Aldrich Nr. 18,464-0)
63) Nylon 6                             (Aldrich Nr. 18,111-0)

**Beispiele 64-66 — Oxidation mit auf einem Polymeren fixierten Methylrheniumtrioxid**

Zu 3 ml der nach B zunächst erhaltenen Oxidationslösung gab man 20 mg des Katalysators nach Beispiel 57. Das unlösliche, katalysatorhaltige Polymere verfärbte sich spontan gelb. Der Suspension wurde unter Rühren das zu oxidierende Olefin zugesetzt, wobei eine Erwärmung beobachtet wurde. Nach 1 Stunde war die gelbe Färbung des Polymeren verschwunden, und das eingestellte Verhältnis Reaktand/Produkt blieb auch bei längerer Wartezeit konstant. Nach weiteren 30 min wurde der Katalysator über eine Glasfritte abfiltriert und dreimal mit je 20 ml Tetrahydrofuran gewaschen. Nach Trocknung im Vakuum der Ölpumpe kann er für weitere Oxidationsansätze verwendet werden. Die Aufarbeitung des Filtrates erfolgte wie für die Beispiele 1-34 beschrieben. Die Ergebnisse sind in Tabelle 3 wiedergegeben.

**Tabelle 3**

| Beispiel/Olefin | Reaktions-bedingungen *) | Produkte | Ausbeute |
|---|---|---|---|
| 64  Cyclohexen | RT, 20 h | Epoxycyclohexan | 27 % |
| 65  Methyloleat | RT, 20 h | 9,10-Epoxyoctadecan-säuremethylester | 85 % |
| 66  Allylalkohol | RT, 20 h | 1,2-Epoxypropan-3-ol | 20 % |

*) RT = Raumtemperatur (ca. 28° C)

**Patentansprüche**

1. Verbindungen der Formel $(R^{11}Re)_k O_l R^{12}{}_m \cdot L_n$ (III), worin bedeuten
$R^{11}$ einen nicht-aromatischen Kohlenwasserstoffrest mit 1 bis 10 C-Atomen oder Aralkyl mit 7 bis 9 C-Atomen, die jeweils durch ein Kohlenstoffatom, an das noch wenigstens ein Wasserstoffatom gebunden ist, an das Rhenium gebunden sind,
$R^{12}$ einen aliphatischen Kohlenwasserstoffrest mit 1 bis 10 C-Atomen, der auch bis zu 3 Siliciumbrückenatome enthalten kann, oder einen Phenylrest, der unsubstituiert ist oder bis zu 3 Substituenten aus der Gruppe Alkyl und/oder Alkoxy mit zusammen höchstens 6 C-Atomen oder bis zu fünf Fluoratome enthält,
L einen Liganden, der über ein bis drei O- und/oder N-Atome an das Rhenium gebunden ist, und die Indizes k 1 oder 2, l eine ganze Zahl von 1 bis 3 · k, m null oder eine ganze Zahl von 1 bis 2 · k und n eine ganze Zahl von 1 bis 3 · k und die Indizes k, l, m und n so gewählt sind, daß ihre Kombination der 5-, 6- oder 7-Wertigkeit des Rheniums gerecht wird.
2. Verbindungen der Formel $(Polymer)_f \cdot (CH_3ReO_3)_g$ (VI), in der die Rheniumverbindung $CH_3ReO_3$ über Amin- oder Amidstickstoff des Polymeren an dieses gebunden ist und in der der Quotient g/f das Gewichtsverhältnis der beiden Komponenten wiedergibt und im Bereich zwischen 0,01 und 0,2, vorzugsweise zwischen 0,02 und 0,1 liegt.

3. Verwendung von Verbindungen der allgemeinen Formel $R^1_a Re_b O_c$ (I), worin a 1 bis 6, b 1 bis 4 und c 1 bis 14 sind und die Summe von a, b und c so ist, daß sie der 5- bis 7-Wertigkeit des Rheniums gerecht wird mit der Maßgabe, daß c nicht größer als 3 · b ist, und worin $R^1$ einen nicht-aromatischen Kohlenwasserstoffrest mit 1 bis 10 C-Atomen oder Aralkyl mit 7 bis 9 C-Atomen darstellt, wobei $R^1$ wenigstens teilweise fluoriert sein kann, die Verbindungen nicht mehr als drei Gruppen mit mehr als 6 C-Atomen je Rheniumatom enthalten und an das C-Atom in α-Stellung noch wenigstens ein Wasserstoffatom gebunden ist, bzw. von Verbindungen der Formel $(R^{11}Re)_k O_l R^{12}_m \cdot L_n$ (III), nach Anspruch 1 bzw. von Verbindungen der Formel $(Polymer)_f \cdot (CH_3 ReO_3)_g$ (VI) nach Anspruch 2 als Katalysatoren zur Oxidation von C-C-Mehrfachbindungen.

4. Verfahren zur Herstellung von Epoxiden durch Oxidation von olefinischen Verbindungen mit Wasserstoffperoxid in Gegenwart von Katalysatoren, und gegebenenfalls weitere Umsetzung zu Diolen, dadurch gekennzeichnet, daß man Olefine der Formel $YCZ=CZ-(CX_2)_n R^2$ (II), worin n null oder eine ganze Zahl von 1 bis 28, X H oder F ist, Y H, ein Kohlenwasserstoffrest mit 1 bis 28 C-Atomen oder $COOR^3$ ist und Z H oder einen aromatischen oder nicht-aromatischen Kohlenwasserstoffrest mit 1 bis 6 C-Atomen darstellt, beide Z aber auch gleichzeitig CO sein können, die in o-Stellung an einen Benzolrest gebunden sind, oder zusammen mit C=C einen cycloaliphatischen Ring bilden, und der Substituent $R^2$ H, Halogen, einen acyclischen, von Dreifachbindungen freien Kohlenwasserstoffrest mit bis zu 24 C-Atomen, der bis zu 5 Doppelbindungen enthalten kann, Aryl mit bis zu 10 C-Atomen, das am Ring 1 bis 3 Substituenten enthalten kann, die unter den angewandten Oxidationsbedingungen unempfindlich sind, ω-Oxoalkenyl, $COOR^3$ oder $OR^4$ ist, worin $R^3$ und $R^4$ Alkyl mit 1 bis 15, vorzugsweise 1 bis 6, Kohlenstoffatomen oder Phenyl bedeutet, das am Ring noch 1 bis 3 Substituenten enthalten kann, die unter den angewandten Oxidationsbedingungen unempfindlich sind, oder worin $R^4$ Trialkylsilyl $R^5_3 Si$ ist und $R^5$ Alkyl mit 1 bis 5, vorzugsweise 1 bis 3 C-Atomen ist, wobei $(CX_2)_n$ und $R^2$ zusammen höchstens 28 C-Atome haben und wobei Y, $(CX_2)_n$ und $R^2$ zusammen mit der Gruppe CZ=CZ auch ein cycloaliphatisches Ringsystem bilden können, das bis zu 5, vorzugsweise bis zu 3 olefinische Bindungen oder bis zu 2 Carbonylgruppen enthalten kann, bei einer Temperatur von −30 bis +80°C, vorzugsweise von 0 bis 40°C, in flüssigem Medium in Gegenwart von Rhenium-Verbindungen der Formel $R^1_a Re_b O_c$ (I), worin a 1 bis 6, b 1 bis 4 und c 1 bis 14 sind und die Summe von a, b und c so ist, daß sie der 5- bis 7-Wertigkeit des Rheniums gerecht wird mit der Maßgabe, daß c nicht größer als 3 · b ist, und worin $R^1$ einen nicht-aromatischen Kohlenwasserstoffrest mit 1 bis 10 C-Atomen oder einen Aralkylrest mit 7 bis 9 C-Atomen darstellt, wobei $R^1$ wenigstens teilweise fluoriert sein kann, die Verbindungen nicht mehr als drei Gruppen mit mehr als 6 C-Atomen je Rheniumatom enthalten und an das C-Atom in α-Stellung noch wenigstens ein Wasserstoffatom gebunden ist, bzw. von Verbindungen der Formel $(R^{11}Re)_k O_l R^{12}_m \cdot L_n$ (III) nach Anspruch 1 bzw. von Verbindungen der Formel $(Polymer)_f \cdot (CH_3 ReO_3)_g$ (VI) nach Anspruch 2 oxidiert und die erhaltenen Epoxide

a) isoliert oder
b) in Gegenwart der gleichen Katalysatoren in 1,2-Diole oder
c) in üblicher Weise mit Basen in 1,2-trans-Diole oder
d) in üblicher Weise mit Säuren in cis-trans-Gemische von 1,2-Diolen umwandelt.

5. Verfahren zur oxidation von Cumulenen der Formel $R^7 C=C=CR^8$ (IV) und von Alkinen der Formel $R^9 C≡CR^{10}$ (V), worin $R^7$ bis $R^{10}$ jeweils Alkyl mit 1 bis 4 C-Atomen oder 1 oder 2 Arylkerne enthaltendes Aryl ist, wobei das Aryl noch Substituenten tragen kann, die ihrerseits unter Oxidationsbedingungen unempfindlich sind, dadurch gekennzeichnet, daß man diese bei einer Temperatur von −30 bis +80°C, vorzugsweise von 0 bis 40°C, in flüssigem Medium mit Wasserstoffperoxid in Gegenwart von Rhenium-Verbindungen der Formel $R^1_a Re_b O_c$ (I), wie im Anspruch 3 definiert, bzw. von Verbindungen der Formel $(R^{11}Re)_k O_l R^{12}_m \cdot L_n$ (III) nach Anspruch 1 bzw. von Verbindungen der Formel $(Polymer)_f \cdot (CH_3 ReO_3)_g$ (VI) nach Anspruch 2 oxidiert.

6. Verfahren zur Oxidation von Olefinen der Formel $R^{13}CH=CHR^{14}$ (VII), worin $R^{13}$ Aryl mit bis zu 10 C-Atomen und $R^{14}$ H, $CH_3$ oder Aryl mit bis zu 10 C-Atomen ist, dadurch gekennzeichnet, daß man diese bei einer Temperatur von −30 bis +80°C, vorzugsweise von 0 bis 40°C, in flüssigem Medium mit Wasserstoffperoxid in Gegenwart von Rhenium-Verbindungen der Formel $R^1_a Re_b O_c$ (I), wie im Anspruch 3 definiert, bzw. von Verbindungen der Formel $(R^{11}Re)_k O_l R^{12}_m \cdot L_n$ (III) nach Anspruch 1 bzw. von Verbindungen der Formel $(Polymer)_f \cdot (CH_3 ReO_3)_g$ (VI) nach Anspruch 2 oxidiert.

7. Verfahren nach einem oder mehreren der Ansprüche 4 bis 6, dadurch gekennzeichnet, daß das Reaktionsmedium aus einer Lösung von Wasserstoffperoxid in tert.-Butanol besteht.

8. Verfahren nach Anspruch 7, dadurch gekennzeichnet, daß die Lösung 1 bis 30% $H_2O_2$ enthält.

9. Verfahren nach einem oder mehreren der Ansprüche 4 bis 8, dadurch gekennzeichnet, daß der Katalysator eine Verbindung der Formel $(CH_3 Re)_k O_l R^{12}_m \cdot L_n$ ist, worin k 1 oder 2, 1 eine ganze Zahl von 1 bis 3 · k, m Null oder eine ganze Zahl von 1 bis 2 · k, n eine ganze Zahl von 1 bis 3 · k und die Indizes k, l, m und n so gewählt sind, daß ihre Kombination der 5-, 6- oder 7-Wertigkeit des Rheniums gerecht wird, $R^{12}$ einen ali-

16

phatischen Kohlenwasserstoffrest mit 1 bis 10 C-Atomen, der auch bis zu 3 Siliziumbrückenatome tragen kann, oder einen Phenylrest, der 1 bis 3 Substituenten der Gruppe Alkyl und/oder Alkoxy mit zusammen höchstens 6 C-Atomen tragen kann, und L einen Lewis-basischen, über ein bis drei O- und/oder N-Atome an das Rhenium gebundenen Liganden bedeutet.

10. Verfahren nach einem oder mehreren der Ansprüche 4 bis 9, dadurch gekennzeichnet, daß der Katalysator $CH_3ReO_3$ in der Form angewendet wird, daß er auf ein Polymeres, das Amin- oder Amid-Stickstoff enthält, aufgezogen ist.

11. Verfahren nach Anspruch 7 oder 8, dadurch gekennzeichnet, daß ein gelöster Katalysator der Formel I gemäß Anspruch 3, worin $R^1CH_3$ ist, verwendet wird und dieser nach erfolgter Verwendung durch Umsetzung mit einem Polymeren, das Amin- bzw. Amid-Stickstoff enthält, aus der Lösung entfernt und entweder

a) als Verbindung der Formel VI gemäß Anspruch 2 erhalten wird oder

b) aus dieser Form durch Behandlung im Hochvakuum bei einer Temperatur von mindestens 200°C als Verbindung der Formel I gemäß Anspruch 3, worin $R^1CH_3$ ist, erhalten wird.

## Claims

1. A compound of the formula $(R^{11}Re)_kO_lR^{12}_m \cdot L_n$ (III) in which

$R^{11}$ is a non-aromatic hydrocarbon radical having 1 to 10 carbon atoms or aralkyl having 7 to 9 carbon atoms which in each case is bonded to the rhenium via a carbon atom to which at least one hydrogen atom has been attached,

$R^{12}$ is an aliphatic hydrocarbon radical having 1 to 10 carbon atoms, which may also contain up to 3 silicon bridge atoms, or a phenyl radical which is unsubstituted or contains up to 3 substituents from the group comprising alkyl and/or alkoxy jointly having at most 6 carbon atoms or contains up to five fluorine atoms,

L is a ligand which is bonded via one to three oxygen atoms and/or nitrogen atoms to the rhenium and

the index k is 1 or 2, the index l is an integer from 1 to 3 times k, the index m is zero or an integer from 1 to 2 times k and the index n is an integer from 1 to 3 times k, and the indices k, l, m and n are selected such that their combination satisfies the 5-, 6- or 7-valence of rhenium.

2. A compound of the formula $(polymer)_f \cdot (CH_3ReO_3)_g$ (VI) in which the rhenium compound $CH_3ReO_3$ is bonded to the polymer via its amine nitrogen or amide nitrogen and in which the quotient g/f expresses the ratio by weight of the two components and is in the range between 0,01 and 0,2, preferably between 0,02 and 0,1.

3. The use of a compound of the formula $R^1_aRe_bO_c$ (I) in which a is 1 to 6, b is 1 to 4 and c is 1 to 14 and the total of a, b and c is such that the 5-valence to 7-valence of rhenium is satisfied, with the proviso that c is not greater then 3 times b, and in which $R^1$ is a non-aromatic hydrocarbon radical having 1 to 10 carbon atoms or aralkyl having 7 to 9 carbon atoms and $R^1$ may be at least partially fluorinated, and the compounds do not contain more than three groups having more than 6 carbon atoms per rhenium atom and the carbon atom in the α-position still has attached to it at least one hydrogen atom, or of a compound of the formula $(R^{11}Re)_kO_lR^{12}_m \cdot L_n$ (III) as claimed in claim 1 or of a compound of the formula $(polymer)_f \cdot (CH_3ReO_3)_g$ (VI) as claimed in claim 2 as a catalyst in the oxidation of multiple C-C bonds.

4. A process for the preparation of epoxides by oxidation of olefinic compounds with hydrogen peroxide in the presence of catalysts, and a further, optional reaction to form diols, which comprises oxidizing olefins of the formula $YCZ=CZ-(CX_2)_nR^2$ (II) in which n is zero or an integer from 1 to 28, X is H or F, Y is H, a hydrocarbon radical having 1 to 28 carbon atoms or $COOR^3$, Z is H or an aromatic or non-aromatic hydrocarbon radical having 1 to 6 carbon atoms, where the two symbols Z may also simultaneously be CO which is bonded in the o-position to a benzene radical, or form a cycloaliphatic ring together with C=C, and the substituent $R^2$ is H, halogen, an acyclic hydrocarbon radical without triple bonds having up to 24 carbon atoms, which may contain up to 5 double bonds, aryl having up to 10 carbon atoms and which may contain 1 to 3 substituents on the ring which are inert to the oxidation conditions used, ω-oxoalkenyl, $COOR^3$ or $OR^4$, where $R^3$ and $R^4$ are alkyl having 1 to 15, preferably 1 to 6, carbon atoms or is phenyl which may contain a further 1 to 3 substituents on the ring which are inert to the oxidation conditions used, or in which $R^4$ is a trialkylsilyl $R^5_3Si$ and $R^5$ is alkyl having 1 to 5, preferably 1 to 3, carbon atoms, and $(CX_2)_n$ and $R^2$ jointly have not more than 28 carbon atoms and Y, $(CX_2)_n$ and $R^2$ together with the group CZ=CZ may also form a cycloaliphatic ring system which can contain up to 5, preferably up to 3, olefinic bonds or up to 2 carbonyl groups, at a temperature of −30 to +80°C, preferably of 0 to 40°C, in a liquid medium in the presence of rhenium compounds of the formula $R^1_aRe_bO_c$ (I) in which a is 1 to 6, b is 1 to 4 and c is 1 to 14 and the total of a, b and c is such that the 5-valence to 7-valence of rhenium is satisfied, with the proviso that c is not greater than 3 times b, and in which $R^1$ is a non-aromatic hydrocarbon radical having 1 to 10 carbon atoms or an aralkyl radical having 7 to 9 carbon atoms and $R^1$ may be at least partially fluorinated, and the compounds do not contain more than three groups having more than

17

6 carbon atoms per rhenium atom and the carbon atom in the a-position still has attached to it at least one hydrogen atom, or of compounds of the formula $(R^{11}Re)_k O_l R^{12}{}_m \cdot L_n$ (III) as claimed in claim 1 or of compounds of the formula $(polymer)_f \cdot (CH_3ReO_3)_g$ (VI) as claimed in claim 2, and

    a) isolating the epoxides obtained or

    b) converting the epoxides obtained in the presence of the same catalysts into 1,2-diols or

    c) converting the epoxides obtained in a customary manner with bases into 1,2-trans-diols or

    d) converting the epoxides obtained in a customary manner with acids into cis-trans mixtures of 1,2-diols.

5. A process for the oxidation of cumulenes of the formula $R^7 C=C=CR^8$ (IV) and of alkynes of the formula $R^9 C\equiv CR^{10}$ (V) in which $R^7$ to $R^{10}$ are in each case alkyl having 1 to 4 carbon atoms or aryl containing 1 or 2 aryl nuclei and the aryl may have other substituents which for their part are inert to oxidation conditions, which comprises oxidizing these compounds with hydrogen peroxide at a temperature of −30 to +80°C, preferably of 0 to 40°C, in a liquid medium in the presence of rhenium compounds of the formula $R^1{}_a Re_b O_c$ (I) as defined in claim 3, or of compounds of the formula $(R^{11}Re)_k O_l R^{12}{}_m \cdot L_n$ (III) as claimed in claim 1, or of compounds of the formula $(polymer)_f \cdot (CH_3ReO_3)_g$ (VI) as claimed in claim 2.

6. A process for the oxidation of olefins of the formula $R^{13}CH=CHR^{14}$ (VII) in which $R^{13}$ is aryl having up to 10 carbon atoms and $R^{14}$ is H, $CH_3$ or aryl having up to 10 carbon atoms, which comprises oxidizing these compounds with hydrogen peroxide at a temperature of −30 to +80°C, preferably of 0 to 40°C, in a liquid medium in the presence of rhenium compounds of the formula $R^1{}_a Re_b O_c$ (I) as defined in claim 3, or of compounds of the formula $(R^{11}Re)_k O_l R^{12}{}_m \cdot L_n$ (III) as claimed in claim 1, or of compounds of the formula $(polymer)_f \cdot (CH_3ReO_3)_g$ (VI) as claimed in claim 2.

7. The process as claimed in one or more of claims 4 to 6, wherein the reaction medium comprises a solution of hydrogen peroxide in tert-butanol.

8. The process as claimed in claim 7, wherein the solution contains 1 to 30% of $H_2O_2$.

9. The process as claimed in one or more of claims 4 to 8, wherein the catalyst is a compound of the formula $(CH_3Re)_k O_l R^{12}{}_m \cdot L_n$ in which k is 1 or 2, l is an integer from 1 to 3 times k, m is zero or an integer from 1 to 2 times k, n is an integer from 1 to 3 times k, and the indices k, l, m and n are selected such that their combination satisfies the 5-, 6- or 7-valence of rhenium, $R^{12}$ is an aliphatic hydrocarbon radical having 1 to 10 carbon atoms which may also carry up to 3 silicon bridge atoms, or a phenyl radical which may carry from 1 to 3 substituents from the group comprising alkyl and/or alkoxy jointly having at most 6 carbon atoms, and L is a Lewis-basic ligand which is bonded via one to three oxygen and/or nitrogen atoms to the rhenium.

10. The process as claimed in one or more of claims 4 to 9, wherein the catalyst $CH_3ReO_3$ is used in the form in which it is taken up on a polymer which contains amine or amide nitrogen.

11. The process as claimed in claim 7 or 8, which comprises using a dissolved catalyst of the formula I as claimed in claim 3 in which $R^1$ is $CH_3$ and, having used this catalyst, removing it from the solution by reacting it with a polymer which contains amine or amide nitrogen and obtaining the catalyst either

    a) as a compound of the formula VI as claimed in claim 2 or

    b) from this form by treatment in high vacuum at a temperature of at least 200°C as a compound of the formula I as claimed in claim 3 in which $R^1$ is $CH_3$.

## Revendications

1. Composés de formule $(R^{11}Re)_k O_l R^{12}{}_m \cdot L_n$ (III), dans laquelle

$R^{11}$ représente un reste d'hydrocarbure non aromatique comportant 1 à 10 atomes de carbone ou aralkyle ayant 7 à 9 atomes de carbone, qui sont fixés sur le rhénium à chaque fois par l'intermédiaire d'un atome de carbone sur lequel est fixé au moins un atome d'hydrogène,

$R^{12}$ représente un reste d'hydrocarbure aliphatique comportant 1 à 10 atomes de carbone, qui peut également contenir jusqu'à 3 atomes de silicium de pontage, ou un reste phényle qui n'est pas substitué ou qui contient jusqu'à trois substituants choisis parmi un reste alkyle et/ou alcoxy ayant ensemble 6 atomes de carbone au maximum ou comportant jusqu'à 5 atomes de fluor,

L représente un ligand ou coordinat, qui est fixé sur le rhénium par l'intermédiaire d'un à trois atomes de O et/ou de N, et

l'indice k vaut 1 ou 2, l'indice 1 est un nombre entier valant 1 à 3 k, m est nul ou est un nombre entier valant 1 à 2 k et n est un nombre entier valant 1 à 3 k, et les indices k, l, m et n sont choisis de manière que leur combinaison corresponde bien à la valence 5, 6 ou 7 du rhénium.

2. Composés de formule $(polymère)_f \cdot (CH_3 ReO_3)_g$ (VI), dans laquelle le composé de rhénium $CH_3 ReO_3$ est fixé sur le polymère par l'intermédiaire d'un azote d'amine ou d'amide de ce polymère et dans laquelle le quotient g/f représente le rapport pondéral des deux constituants et se situe dans la gamme comprise entre 0,01 et 0,2, avantageusement entre 0,02 et 0,1.

3. Utilisation de composés de formule générale $R^1_a Re_b O_c$ (I) (dans laquelle a vaut 1 à 6, b vaut 1 à 4 et c vaut 1 à 14, et la somme de a, b et c est telle qu'elle correspond à la valence 5 à 7 du rhénium, à la condition que le nombre c ne soit pas supérieur à 3 b ; et $R^1$ représente un reste d'hydrocarbure non aromatique comportant 1 à 10 atomes de carbone ou aralkyle ayant 7 à 9 atomes de carbone, le groupe $R^1$ pouvant au moins partiellement être fluoré, les composés ne contiennent pas plus de trois groupes comportant plus de 6 atomes de carbone par atome de rhénium, et, sur l'atome de C en position alpha, il y a encore au moins un atome d'hydrogène qui est fixé, ou utilisation de composés de formule $(R^{11}Re)_k O_l R^{12}_m \cdot L_n$ (III), selon la revendication 1 ou de composés de formule $(polymère)_f \cdot (CH_3ReO_3)_g$ (VI) selon la revendication 2, comme catalyseurs pour l'oxydation de liaisons multiples C-C.

4. Procédé pour préparer des époxydes par l'oxydation de composés oléfiniques à l'aide de peroxyde d'hydrogène en présence de catalyseurs et éventuellement pour la poursuite de la réaction donnant des diols, procédé caractérisé en ce qu'on oxyde des oléfines de formule $YC=CZ-(CX_2)_n R^2$ (II), dans laquelle n est nul ou représente un nombre entier valant 1 à 28 ; X représente H ou F ; Y représente H, un reste d'hydrocarbure ayant 1 à 28 atomes de carbone ou $COOR^3$, et Z représente H ou un reste d'hydrocarbure aromatique ou non aromatique ayant 1 à 6 atomes de carbone, les deux Z pouvant cependant aussi représenter simultanément CO qui sont fixés en position ortho sur un reste benzénique ou pouvant former, avec C=C un noyau cycloaliphatique, et le substituant $R^2$ représente un atome d'H ou d'halogène, un reste d'hydrocarbures acycliques dépourvus de triples liaisons et comportant jusqu'à 24 atomes de carbone, qui peut contenir jusqu'à cinq doubles liaisons, un reste aryle ayant jusqu'à 10 atomes de carbone, qui peut contenir sur le noyau un à trois substituants insensibles aux conditions appliquées pour l'oxydation, un reste oméga-oxoalcényle, $COOR^3$ ou $OR^4$, où $R^3$ et $R^4$ représentent chacun un reste alkyle ayant 1 à 15, avantageusement 1 à 6, atomes de carbone ou un reste phényle qui peut contenir sur le noyau encore un à trois substituants insensibles aux conditions appliquées pour l'oxydation, ou bien $R^4$ représente un reste trialkylsilyle $R^5_3Si$ et $R^5$ représente un reste alkyle ayant 1 à 5, avantageusement 1 à 3 atomes de carbone, le groupe $(CX_2)_n$ et $R^2$ comportant ensemble 28 atomes de C au maximum, et Y, $(CX_2)_n$ et $R^2$ pouvant former avec le groupe CZ=CZ également un système cyclique cycloaliphatique pouvant contenir jusqu'à 5, avantageusement jusqu'à 3 liaisons oléfiniques ou jusqu'à deux groupes carbonyles), à une température de $-30$ jusqu'à $+80°C$, avantageusement de 0 à 40°C, en milieu liquide en présence de composés du rhénium de formule $R^1_a Re_b O_c$ (I), (dans laquelle a vaut 1 à 6, b vaut 1 à 4 et c vaut 1 à 14, et la somme de a, b et c est telle qu'elle correspond à la valence 5 à 7 du rhénium, à la condition que c ne vale pas plus de 3 b, et $R^1$ représente un reste d'hydrocarbure non aromatique comportant 1 à 10 atomes de carbone, ou un reste aralkyle ayant 7 à 9 atomes de carbone, le reste $R^1$ pouvant être au moins partiellement fluoré, les composés ne contenant pas plus de trois groupes ayant plus de 6 atomes de carbone par atome de rhénium et, sur l'atome de carbone en position alpha, il y a eu moins un atome d'hydrogène fixé), ou en présence de composés de formule $(R^{11}Re)_k O_l R^{12}_m \cdot L_n$ (III) selon la revendication 1 ou de composés de formule $(polymère)_f \cdot (CH_3ReO_3)_g$ (VI) selon la revendication 2, et l'on soumet les époxydes ainsi obtenus ;

a) à un isolement, ou

b) à une transformation, en présence des mêmes catalyseurs, en des diols-1,2, ou

c) de façon usuelle, à l'aide de bases, à une transformation en des diols trans-1,2, ou

d) de façon usuelle, à l'aide d'acides, en des mélanges cis-trans de diols-1,2.

5. Procédé pour oxyder des cumulènes de formule $R^7C=C-CR^8$ (IV) et des alcynes de formule $R^9C\equiv CR^{10}$ (V), (formules dans lesquelles $R^7$ à $R^{10}$ représentent chacun un reste alkyle ayant 1 à 4 atomes de carbone ou un reste aryle contenant un ou deux noyaux aryles, le reste aryle pouvant encore porter les substituants qui sont pour leur part insensibles aux conditions d'oxydation), procédé caractérisé en ce qu'on oxyde les cumulènes à une température de $-30$ jusqu'à $+80°C$, avantageusement de 0 à 40°C, en milieu liquide, avec du peroxyde d'hydrogène en présence de composés du rhénium de formule $R^1_a Re_b O_c$ (I), selon la définition indiquée à la revendication 3, ou en présence de composés de formule $R^{11}Re)_k O_l R^{12}_m \cdot L_n$ (III) selon la revendication 1, ou en présence de composés de formule $(polymère)_f \cdot (CH_3ReO_3)_9$ (VI) selon la revendication 2.

6. Procédé pour oxyder des oléfines de formule $R^{13}CH=CHR^{14}$ (VII), (dans laquelle $R^{13}$ représente un reste aryle ayant jusqu'à 10 atomes de carbone et $R^{14}$ représente H, $CH_3$ ou un reste aryle ayant jusqu'à 10 atomes de carbone), caractérisé en ce qu'on oxyde ces oléfines, à une température allant de $-30$ jusqu'à $+80°C$, avantageusement entre 0 et 40°C, en milieu liquide avec du peroxyde d'hydrogène, en présence de composés du rhénium de formule $R^1_a Re_b O_c$ (I), selon la définition de la revendication 3 ou en présence de composés de formule $(R^{11}Re)_k O_1 R^{12}_m \cdot L_n$ (III) selon la revendication 1 ou en présence de composés de formule $(polymère)_f \cdot (CH_3 ReO_3)_g$ (VI) selon la revendication 2.

7. Procédé selon une ou plusieurs des revendications 4 à 6, caractérisé en ce que le milieu de réaction consiste en une solution du peroxyde d'hydrogène dans du tertiobutanol.

8. Procédé selon la revendication 7, caractérisé en ce que la solution contient 1 à 30% de $H_2O_2$.

9. Procédé selon une ou plusieurs des revendications 4 à 8, caractérisé en ce que le catalyseur est un composé de formule $(CH_3Re)_kO_lR^{12}_m \cdot L_n$ (dans laquelle k vaut 1 ou 2 ; 1 est un nombre entier valant 1 à 3 k ; m est nul ou est un nombre entier valant 1 à 2 k ; n est un nombre entier valant 1 à 3 k et les indices k, l, m et n sont choisis de manière que leurs combinaisons correspondent à la valence 5, 6 ou 7 du rhénium ; $R^{12}$ représente un reste d'hydrocarbure aliphatique ayant 1 à 10 atomes de carbone, qui peut également porter jusqu'à 3 atomes de silicium de pontage, ou un reste phényle, qui peut porter un à trois substituants choisis parmi un reste alkyle et/ou alcoxy comportant ensemble 6 atomes de carbone au maximum ; et L représente un ligand ou coordinat, qui est une base de Lewis et qui est fixé sur le rhénium par l'intermédiaire d'1 à 3 atomes de O et/ou de N).

10. Procédé selon une ou plusieurs des revendications 4 à 9, caractérisé en ce qu'on applique le catalyseur $CH_3ReO_3$ sous la forme d'une matière revêtant un polymère, lequel contient de l'azote d'amine ou d'amide.

11. Procédé selon la revendication 7 ou 8, caractérisé en ce qu'on utilise un catalyseur dissous, de formule I selon la revendication 3 (dans laquelle $R^1$ représente $CH_3$) et, après son utilisation, on retire ce catalyseur de la solution en le faisant réagir avec un polymère contenant de l'azote d'amine ou d'amide, et

a) on l'obtient sous forme d'un composé de formule (VI) selon la revendication 2, ou

b) à partir de cette forme, on l'obtient, par traitement sous vide poussé à une température d'au moins 200°C, sous forme d'un composé de formule I selon la revendication 3, dans laquelle $R^1$ représente $CH_3$.